# EUROPÄISCHE PATENTSCHRIFT

(11) **EP 0 405 704 B1**
(45) Veröffentlichungstag und Bekanntmachung des Hinweises auf die Patenterteilung: **14.12.1994**
(21) Anmeldenummer: 90250165.9
(22) Anmeldetag: 27.06.1990
(51) Int. Cl.: C07C 229/04, C07C 227/18, A61K 31/195, A61K 49/00

(54) **Derivatisierte DTPA-Komplexe, diese Verbindungen enthaltende pharmazeutische Mittel, ihre Verwendung und Verfahren zu deren Herstellung**
DTPA-complexes derivatives, pharmaceutical compositions containing them, their use and process for their preparation
Dérivés de DTPA-complèxes, compositions pharmaceutiques les contenant, leur utilisation et procédé pour leur fabrication

(30) Priorität: 30.06.1989 DE 3922005
(43) Veröffentlichungstag der Anmeldung: 02.01.1991
(73) Patentinhaber: SCHERING AKTIENGESELLSCHAFT, 13342 Berlin (DE)
(72) Erfinder: Schmitt-Willich, Heribert, Dr., D-1000 Berlin 45 (DE); Platzek, Johannes, Dr., D-1000 Berlin 33 (DE); Gries, Heinz, Dr., D-1000 Berlin 31 (DE); Schuhmann-Giampieri, Gabriele, Dr., D-1000 Berlin 45 (DE); Vogler, Hubert, Dr., D-1000 Berlin 12 (DE); Weinmann, Hanns-Joachim, Dr., D-1000 Berlin 38 (DE)

(56) Entgegenhaltungen:
- EP-A- 0 165 716
- EP-A- 0 263 059
- EP-A- 0 299 795
- EP-A- 0 305 320
- EP-A- 0 315 220
- WO-A-88/07521
- DE-A- 3 401 052

## Beschreibung

Die Erfindung betrifft den in den Patentansprüchen gekennzeichneten Gegenstand, das heißt neue Komplexe und Komplexsalze, diese Verbindungen enthaltende Mittel, ihre Verwendung für die Herstellung von Mitteln für die NMR- und Röntgen-Diagnostik und Strahlentherapie sowie Verfahren zur Herstellung dieser Verbindungen und Mittel.

Metallkomplexe sind schon zu Beginn der 50er Jahre als Kontrastmittel für die Radiologie in Betracht gezogen worden. Die damals eingesetzten Verbindungen waren aber derart toxisch, daß eine Verwendung beim Menschen nicht in Betracht kam. Es war daher durchaus überraschend, daß sich bestimmte Komplexsalze als ausreichend verträglich erwiesen haben, so daß eine routinemäßige Anwendung am Menschen für diagnostische Zwecke in Erwägung gezogen werden konnte. Als erster Vertreter dieser Substanzklasse hat sich das in der europäischen Patentanmeldung mit der Publikationsnummer 71564 beschriebene Dimegluminsalz des Gd DTPA (Gadolinium-III-Komplex der Diethylentriaminpentaesslgsäure) als Kontrastmittel für die Kernspintomographie sehr gut bewährt. Es ist unter dem Namen Magnevist^{(R)} weltweit als erstes NMR-Diagnostikum registriert worden.

Magnevist^{(R)} ist besonders gut für die Diagnose pathologischer Bereiche (z.B. Entzündungen, Tumore, Infarkte etc.) geeignet. Nach intravenöser Injektion verteilt sich die Verbindung extrazellulär und wird durch glomeruläre Sekretion über die Nieren eliminiert. Eine Passage intakter Zellmembranen und eine extrarenale Ausscheidung werden praktisch nicht beobachtet.

Besonders für Patienten mit eingeschränkter Nierenfunktion, bei denen Magnevist^{(R)} nur sehr langsam ausgeschieden wird und zum Teil nur mit Hilfe eines Dialvsegerätes aus dem Organismus entfernt werden kann, wären Kontrastmittel, die eine zumindest nur teilweise extrarenale Ausscheidung aufweisen, wünschenswert.

Es besteht daher ein Bedarf an NMR-Kontrastmitteln, die ein anderes pharmakokinetisches Verhalten als Magnevist^{(R)} zeigen.

Der Erfindung liegt somit die Aufgabe zugrunde, derartige Verbindungen und Mittel zur Verfügung zu stellen sowie ein Verfahren zu ihrer Herstellung zu schaffen. Diese Aufgabe wird durch die vorliegende Erfindung erfüllt.

Die erfindungsgemäßen Verbindungen zeigen die gewünschte Eigenschaft: sowohl renale Ausscheidung als auch Exkretion mit den Faeces.

Überraschenderweise ist die Elimination über die Galle jedoch nicht der einzige extrarenale Ausscheidungsweg: bei NMR-Untersuchungen an Ratten wurde nach intravenöser Gabe der erfindungsgemäßen Verbindungen unerwartet auch eine Kontrastverstärkung des Magen-Darm-Traktes beobachtet. Die Nieren sowie implantierte Tumore werden ebenfalls besser kontrastiert.

Die Ausscheidung (Sekretion) über den Magen hat den Vorteil, daß eine Abgrenzung abdominaler Strukturen (z.B. Pankreas) vom Gastrointestinal-Trakt mit gleichzeitiger Kontrastverstärkung pathologischer Prozesse (Tumoren, Entzündungen) ermöglicht wird. Eine Darstellung des renalen Systems, der Leber und der Gallenblase und Gallenwege kann darüber hinaus auch erzielt werden. Neben der verbesserten Darstellung von Ulci und Magenkarzinomen kann auch eine Überprüfung der Magensaftsekretion mit Hilfe bildgebender Verfahren durchgeführt werden.

Durch die Zurverfügungstellung der erfindungsgemäßen Verbindungen kann somit sowohl niereninsuffizienten als auch den unter gastrointestinalen Erkrankungen leidenden Patienten (mindestens 10 % der Bevölkerung in den westlichen Industrieländern) geholfen werden. Die meisten dieser Patienten sowie eine große Anzahl von Patienten, bei denen der Verdacht auf eine solche Erkrankung vorliegt, müssen sich diagnostischen Untersuchungen unterziehen. Zur Zeit sind vor allem zwei dafür geeignete Methoden gebräuchlich: Die Endoskopie und die Röntgenstrahl-Diagnostik mit Hilfe von Barium-Kontrastmitteln.

Diese Untersuchungen weisen verschiedene Nachteile auf: sie sind mit dem Risiko der Strahlenbelastung behaftet, trauma-verursachend, mit Unannehmlichkeiten, gelegentlich sogar mit Risiken für den Patienten verbunden und können daher psychologischen Streß hervorrufen. Sie müssen meist wiederholt durchgeführt werden, sind relativ aufwendig durchzuführen, erfordern die aktive Mitarbeit des Patienten (z.B. Einnehmen einer bestimmten Körperhaltung) und sind oft nicht anwendbar bei gebrechlichen und bei Risiko-Patienten.

Die Aufgabe, neue diagnostische Methoden zur Erkennung und Lokalisierung gastrointestinaler Erkrankungen, die diese Nachteile nicht besitzen, zur Verfügung zu stellen, wird daher durch die erfindungsgemäßen Komplexverbindungen und Mittel ebenfalls gelöst.

Auch ohne spezifische Maßnahmen erlaubt ihre Pharmakokinetik die Verbesserung der Diagnose zahlreicher Erkrankungen. Die Komplexe werden zum größten Teil unverändert und rasch wieder ausgeschieden, so daß insbesondere auch im Falle der Verwendung relativ toxischer Metallionen auch bei hoher Dosierung keine schädlichen Wirkungen beobachtet werden.

Die praktische Anwendung der neuen Komplexe wild auch durch deren günstige chemische Stabilität erleichtert.

Die in WO-A-88/07521 und EP-A-O 299 795 offenbarten Metallkomplexe sind ebenso wie Magnevist® extrazelluläre Kontrastmittel und zeigen daher ein Magnevist® vergleichbares pharmakokinetisches Verhalten. Insbesondere weisen die erfindungsgemäßen Verbindungen im Vergleich zu diesen vorbekannten Metallkomplexen überraschenderweise eine deutlich höhere Exkretion mit den Faeces auf.

Die in EP-A-0 165 716 und EP-A-0 315 220 genannten, überwiegend höhermolekularen Verbindungen sind Metallkomplexe, die - im Unterschied zu den erfindungsgemäßen Verbindungen - an ein organisches Spezies wie ein Protein, Steroid, Peptid, Kohlenwasserstoff oder Pharmakon chemisch gebunden sind. Es findet sich in diesen Dokumenten kein Hinweis darauf, daß mit diesen Verbindungen die dieser Erfindung zingrunde liegenden Aufgaben gelöst werden können.

Die Erfindung betrifft daher die Verwendung von mindestens einer physiologisch verträglichen Verbindung der allgemeinen Formel I 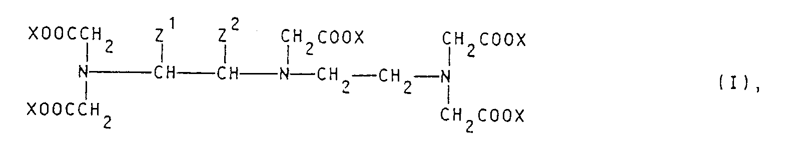
worin
- Z¹ und Z²: unabhängig voneinander für ein Wasserstoffatom oder den Rest -(CH₂)ₘ-(C₆H₄)_{q}-(O)ₖ-(CH₂)ₙ-(C₆H₄)ₗ-(O)ᵣ-R,
worin
m und n die Ziffern 0 - 20,
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms, eines C₁-C₆-Alkylrestes oder einer Benzylgruppe bedeuten,
- X: für ein Wasserstoffatom und/oder ein Metallionenäquivalent eines Elements der Ordnungszahlen 21 - 29, 42, 44 oder 57 - 83 stehen, mit der Maßgabe, daß mindestens zwei der Substituenten X für ein Metallionenäquivalent stehen, daß einer der Substituenten Z¹ und Z² für ein Wasserstoffatom und der andere nicht für ein Wasserstoffatom steht, daß - wenn n und l jeweils für die Ziffer 0 stehen -
k und r nicht gleichzeitig jeweils die Ziffer 1 bedeuten, daß Z¹ oder Z² nicht für -CH₂-C₆H₄-O-CH₂-COOCH₂C₆H₅ oder -CH₂-C₆H₄-O-(CH₂)₅-COOCH₂C₆H₅ stehen, daß -(O)ᵣ-R nicht für -OH steht, daß Z¹ nicht für 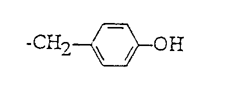 steht und daß die Summe von q und l die Ziffer 1 oder 2 ergibt, sowie deren Salze mit anorganischen und/oder organischen Basen, Aminosäuren oder Aminosäureamiden für die Herstellung von Mitteln für die NMR-Diagnostik des Magen-Darm-Traktes.

Die erfindungsgemäßen Verbindungen werden durch die allgemeine Formel I gekennzeichnet 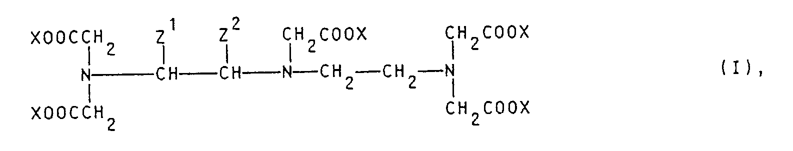
worin
- Z¹ und Z²: unabhängig voneinander für ein Wasserstoffatom oder den Rest -(CH₂)ₘ-(C₆H₄)_{q}-(O)ₖ-(CH₂)ₙ-(C₆H₄)ₗ-(O)ᵣ-R,
worin
m und n die Ziffern 0 - 20,
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms, eines C₁-C₆-Alkylrestes oder einer Benzylgruppe bedeuten,
- X: für ein Wasserstoffatom und/oder ein Metallionenäquivalent eines Elements der Ordnungszahlen 21 - 29, 42, 44 oder 57 - 83 stehen, mit der Maßgabe, daß mindestens zwei der Substituenten X für ein Metallionenäquivalent stehen, daß einer der Substituenten Z¹ und Z² für ein Wasserstoffatom und der andere nicht für ein Wasserstoffatom steht, daß - wenn n und l jeweils für die Ziffer 0 stehen -
k und r nicht gleichzeitig jeweils die Ziffer 1 bedeuten, daß Z¹ oder Z² nicht für -CH₂-C₆H₄-O-CH₂-COOCH₂C₆H₅ oder -CH₂-C₆H₄-O-(CH₂)₅-COOCH₂C₆H₅ stehen, daß -(O)ᵣ-R nicht für -OH steht, daß Z¹ nicht für 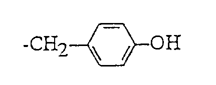 steht und daß die Summe von q und l die Ziffer 1 oder 2 ergibt, sowie deren Salze mit anorganischen und/oder organischen Basen, Aminosäuren oder Aminosäureamiden.

Falls das erfindungsgemäße Mittel zur Anwendung in der NMR-Diagnostik bestimmt ist, muß das Zentralion des Komplexsalzes paramagnetisch sein. Dies sind insbesondere die zwei- und dreiwertigen Ionen der Elemente der Ordnungszahlen 21-29, 42, 44 und 58-70. Geeignete Ionen sind beispielsweise das Chrom(III)-, Mangan(II)-, Eisen(II)-, Cobalt(II)-, Nickel(II)-, Kupfer(II)-, Praseodym(III)-, Neodym(III)-, Samarium(III)- und Ytterbium(III)-ion. Wegen ihres sehr starken magnetischen Moments sind besonders bevorzugt das Gadolinium(III)-, Terbium(III)-, Dysprosium(III)-, Holmium(III)-, Erbium(III)- und Eisen(III)-ion.

Ist das erfindungsgemäße Mittel zur Anwendung in der Röntgen-Diagnostik bestimmt, so muß sich das Zentralion von einem Element höherer Ordnungszahl ableiten, um eine ausreichende Absorption der Röntgenstrahlen zu erzielen. Es wurde gefunden, daß zu diesem Zweck diagnostische Mittel, die ein physiologisch verträgliches Komplexsalz mit Zentralionen von Elementen der Ordnungszahlen zwischen 21-29, 42, 44, 57-83 enthalten, geeignet sind; dies sind beispielsweise das Lanthan(III)-ion und die oben genannten Ionen der Lanthanidenreihe.

Die für m und n stehenden Ziffern sind bevorzugt 0 bis 5.

Als Alkylsubstituenten R und R¹ kommen geradkettige oder verzweigte Kohlenwasserstoffe mit bis zu 6, bevorzugt bis zu 4, Kohlenstoffatomen, die im Falle von R gegebenenfalls durch eine oder mehrere, bevorzugt 1 bis 3, Hydroxy- oder C₁-C₆-, bevorzugt C₁-C₄-Alkoxygruppen substituiert sind, infrage.

Als gegebenenfalls substituierte Alkylgruppen seien beispielsweise die Methyl-, Hydroxymethyl-, Ethyl-, 2-Hydroxyethyl-, 2-Hydroxy-1-(hydroxymethyl)-ethyl-, 1-(Hydroxymethyl)-ethyl-, Propyl-, Isopropyl-, 2- und 3-Hydroxypropyl, 2,3-Dihydroxypropyl-, n-, sek.- und tert.-Butyl-, 2-, 3-, und 4-Hydroxybutyl-, 2- und 3-Hydroxy-isobutyl, Pentyl-, 2- ,3- und 4-Hydroxy-2-methylbutyl-, 2,3,4-Trihydroxybutyl-, 1,2,4-Trihydroxybutyl-, Cyclopentyl-, Cyclohexyl-, 2,3,4,5,6-Pentahydroxyhexylgruppe sowie - im Falle der Hydroxyalkylgruppen - deren C₁-C₆-, bevorzugt C₁-C₄-Alkylderivate, genannt.

Bevorzugte Substituenten Z¹ bzw. Z² der erfindungsgemäßen Verbindungen sind der CH₂-C₆H₄-OCH₃-, -CH₂-C₆H₅-, -CH₂-C₆H₄-O-CH₂-C₆H₄-OCH₃-, -CH₂-O-CH₂-C₆H₅-, -CH₂-C₆H₄-O-CH₂-COOH-, -CH₂-C₆H₄-OC₂H₅-, -CH₂-C₆H₄-OC₄H₉-, -CH₂-C₆H₄-O-CH₂-C₆H₅-Rest.

Als zusätzlicher bevorzugter Substituent Z¹ bzw. Z² für die Verwendung als Mittel für die NMR-Diagnostik des Magen-Darm-Traktes sei der -CH₂-C₆H₄OH-Rest genannt.

Wenn nicht alle aciden Wasserstoffatome durch das Zentralion substituiert werden, können ein, mehrere oder alle verbleibenden Wasserstoffatom(e) durch Kationen anorganischer und/oder organischer Basen oder Aminosäuren ersetzt sein. Geeignete anorganische Kationen sind beispielsweise das Lithiumion, das Kaliumion, das Calciumion, das Magnesiumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins. Geeignete Kationen von Aminosäureamiden sind beispielsweise die des Lysinmethylamids, Glycinethylamids und des Serinmethylamids.

Die Herstellung der erfindungsgemäßen Komplexverbindungen der allgemeinen Formel I erfolgt dadurch, daß man in an sich bekannter Weise Verbindungen der allgemeinen Formel II 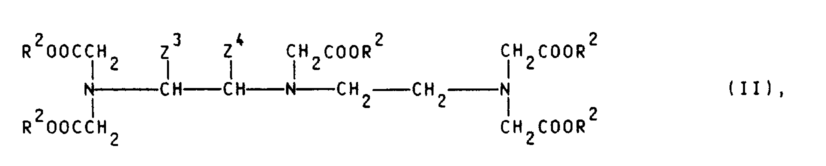
worin
- R²: für eine Säureschutzgruppe,
- Z³ und Z⁴: jeweils für ein Wasserstoffatom oder den Rest -(CH₂)ₘ-(C₆H₄)_{q}-OH, mit der Maßgabe, daß einer der Substituenten Z³ und Z⁴ für ein Wasserstoffatom und der andere für den angegebenen Rest steht,
stehen,
in eine Verbindung mit dem für Z¹ und Z² angegebenen Rest umwandelt, die Säureschutzgruppen R² abspaltet, die so erhaltenen Komplexbildner-Säuren der allgemeinen Formel I mit X in der Bedeutung eines Wasserstoffatoms (Formel I' ) mit mindestens einem Metalloxid oder Metallsalz eines Elements der Ordnungszahlen 21-29, 42, 44 oder 57-83 umsetzt und anschließend - falls gewünscht - vorhandene acide Wasserstoffatome durch Kationen anorganischer und/oder organischer Basen, Aminosäuren oder Aminosäureamide substituiert.

Als Säureschutzgruppen R² kommen niedere Alkyl-, Aryl- und Aralkylgruppen, beispielsweise die Methyl-, Ethyl-, Propyl-, n-Butyl-, t-Butyl-, Phenyl-, Benzyl-, Diphenylmethyl-, Triphenylmethyl-, bis(p-Nitrophenyl)-methylgruppe, sowie Trialkylsilylgruppen infrage.

Die Abspaltung der Schutzgruppen R² erfolgt nach den dem Fachmann bekannten Verfahren [z.B. E. Wünsch, Methoden der Org. Chemie (Houben-Weyl), Bd. XV/1, 4. Aufl. 1974, S 315 ff] beispielsweise durch Hydrolyse, Hydrogenolyse oder alkalische Verseifung der Ester mit Alkali in wäßrig-alkoholischer Lösung bei Temperaturen von 0 bis 50 °C. Zur Abspaltung der für die vorliegenden Reaktionen besonders vorteilhaften t-Butylester werden organische oder anorganische Säuren verwendet: Die in einem geeigneten wasserfreien organischen Lösungsmittel gelöste Esterverbindung, vorzugsweise jedoch die gepulverte Trockensubstanz, wird entweder mit Halogenwasserstoff-Lösung in Eisessig, mit Trifluoressigsäure oder auch Bortrifluorid-diethyletherat in Eisessig versetzt und bei Temperaturen von -10 °C bis 60 °C, vorzugsweise bei Raumtemperatur, abgespalten.

Die als Edukte für die Herstellung der erfindungsgemäßen Komplexverbindungen dienenden Verbindungen der allgemeinen Formel II sind bekannt (DOS 3 710 730 und dort zitierte Literatur) oder können analog der dort beschriebenen Herstellungsvorschriften synthetisiert werden.

Für die Umsetzung der bekannten aliphatischen oder aromatischen Hydroxyverbindungen zu den entsprechenden Aryl-alkyl- bzw. Dialkylethern steht eine Reihe von dem Fachmann bekannten Literaturmethoden zur Verfügung (z.B. J. March, Advanced Organic Chemistry, third edition 1985. S. 342 ff).

Dazu werden die Verbindungen der Formel II, wobei R² für eine alkali-stabile Säureschutzgruppe steht, in einem polar aprotischen Lösungsmittel, wie z.B. Tetrahydrofuran, Dimethoxyethan oder Dimethylsulfoxid, gelöst und mit einer Base, wie z.B. Natriumhydrid, Natriumhydroxid oder Alkali- oder Erdalkalicarbonaten, bei Temperaturen zwischen -30 °C und dem Siedepunkt des jeweiligen Lösungsmittels versetzt, vorzugsweise jedoch zwischen 0 °C und 60 °C.

Dazu wird eine Verbindung der allgemeinen Formel III

Y-(CH₂)ₙ-(C₆H₄)ₗ- (O)ᵣ-R (III)

gegeben, wobei Y für ein Nucleofug, wie z.B. Cl, Br, I, CH₃-C₆H₄SO₃ oder CF₃SO₃, steht und die übrigen Indices die gleiche Bedeutung haben wie in der allgemeinen Formel I.

Die Reaktionszeiten betragen je nach sterischer Hinderung der beteiligten Reste 30 Minuten bis 8 Stunden.

Alternativ zu den oben beschriebenen Reaktionsbedingungen können sowohl Aryl-alkyl- als auch Dialkylether sehr vorteilhaft durch Phasen-Transfer-Katalyse (Starks and Liotta, Phase Transfer Catalysis, Academic Press, N.Y. 1978, S. 128-138) hergestellt werden.

Dazu wird die Reaktion in einem Zweiphasengemisch aus wäßriger Base, vorzugsweise 30 %iger Natronlauge, und einem mit Wasser nicht mischbaren organischen aprotischen Lösungsmittel durchgeführt. Als Phasen-Transferkatalysatoren kommen die dem Fachmann bekannten Verbindungen infrage, vorzugsweise jedoch Tetraalkylammonium- oder Tetraalkylphosphoniumsalze.

Will man Verbindungen der allgemeinen Formel I mit k, n, l und r = O und R in der Bedeutung eines Wasserstoffatoms synthetisieren, so ist es möglich, von der entsprechenden unsubstituierten Aminosäure (z.B. Phenylalanin) ausgehend, die Synthese analog den literaturbekannten Methoden durchzuführen.

Soll jedoch eine Reihe analoger Verbindungen synthetisiert werden, empfiehlt sich die Darstellung der in DOS 3 710 730 beschriebenen Phenolderivate und die reduktive Entfernung der Phenolfunktion nach den dem Fachmann bekannten Literaturverfahren. Genannt sei vor allem die Reduktion von Aryl-diethylphosphaten mit Titan, die sich auch in Gegenwart von Estergruppen sehr vorteilhaft durchführen läßt [S.C. Welch et al. , J. Org. Chem 43, 4797-99 (1978) und dort zitierte Literatur]. Hierbei wird zunächst aus dem phenolischen Edukt durch Umsetzung mit Phosphorsäure-diethylesterchlorid in 70 - bis 100 %iger Ausbeute das entsprechende Aryl-diethylphosphat gebildet, vorzugsweise durch Verwendung von Natriumhydrid als Base in einem polar aprotischen Lösungsmittel. Anschließend wird die Reduktion mit frisch hergestelltem Titanmetall durchgeführt. Vorzugsweise wird zur Herstellung von hochaktivem Titan wasserfreies Titan(III)-chlorid durch Magnesium oder Kalium in wasserfreiem Tetrahydrofuran unter Inertgas reduziert.

Zu einer solchen Mischung wird das oben beschriebene Diethylphosphat zugegeben und 2 bis 24 Stunden, vorzugsweise 6 bis 16 Stunden, unter Rückfluß erhitzt. Nach Beendigung der Reaktion wird gegebenenfalls chromatographisch aufgearbeitet. Ebenfalls anwendbar ist die Palladium-katalysierte Reduktion der entsprechenden Aryl-Triflate nach S. Cacchi et al. , Tetr. Lett. 27, 5541-44 (1986).

Die so erhaltenen Verbindungen der allgemeinen Formel I' mit X in der Bedeutung eines Wasserstoffatoms stellen Komplexbildner dar. Sie können isoliert und gereinigt werden oder ohne Isolierung in Metallkomplexe der allgemeinen Formel I mit mindestens zwei der Substituenten X in der Bedeutung eines Metallionenäquivalents überführt werden.

Die Herstellung der erfindungsgemäßen Metallkomplexe erfolgt in der Weise, wie sie in der Patentschrift DE 3 401 052 offenbart worden ist, indem man das Metalloxid oder ein Metallsalz (beispielsweise das Nitrat, Acetat, Carbonat, Chlorid oder Sulfat) des Elements der Ordnungszahlen 21 - 29, 42, 44 oder 58 - 70 in Wasser und/oder einem niederen Alkohol (wie Methanol, Ethanol oder Isopropanol) löst oder suspendiert und mit der Lösung oder Suspension der äquivalenten Menge der komplexbildenden Säure der allgemeinen Formel I' mit X in der Bedeutung eines Wasserstoffatoms umsetzt, vorzugsweise bei Temperaturen zwischen 40 und 100 °C, und anschließend - falls gewünscht - vorhandene acide Wasserstoffatome von Säuregruppen durch Kationen anorganischer und/oder organischer Basen, Aminosäuren oder Aminosäureamide substituiert.

Die Neutralisation erfolgt dabei mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen, wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Ornithin.

Zur Herstellung der neutralen Komplexverbindungen kann man beispielsweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und anderen), niederen Ketonen (Aceton und andere), polaren Ethern (Tetrahydrofran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel Diethylentriaminpentaessigsäure) oder, falls erforderlich, Elektrolyte (wie zum Beispiel Natriumchlorid) oder, falls erforderlich, Antioxidantien wie zum Beispiel Ascorbinsäure.

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) zum Beispiel Lecithine, Tween^{(R)}, Myrj^{(R)} und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel werden in einer Dosis von 1 µmol/kg bis 5 mmol/kg, vorzugsweise von 10 µmol bis 0,5 mmol/kg des erfindungsgemäßen Komplexsalzes appliziert. Bei einer intravenösen Injektion finden wäßrige Formulierungen der Konzentration 50 µmol/l bis 2 mol/l, vorzugsweise 100 mmol/l bis 1 mol/l, Verwendung. Eine rektale sowie orale Anwendung wird vorzugsweise mit Lösungen der Konzentration 0,1 mmol/l bis 100 mmol/l durchgeführt. Die applizierten Volumina sind je nach diagnostischer Fragestellung zwischen 5 ml und 2 l.

Die erfindungsgemäßen Mittel erfüllen die vielfältigen Voraussetzungen für die Eignung als Kontrastmittel. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität in-vitro auf, sondern auch eine überraschend hohe Stabilität in-vivo, so daß eine Freigabe oder ein Austausch der in den Komplexen nicht konvalent gebundenen - an sich giftigen - Ionen innerhalb der Zeit, in der die neuen Kontrastmittel vollständig wieder ausgeschieden werden, nur äußerst langsam erfolgt.

Die erfindungsgemäßen Mittel können auch für die Strahlentherapie verwendet werden. So sind Komplexe des Gadoliniums aufgrund des großen Einfangquerschnitts für die Neutroneneinfangtherapie hervorragend geeignet. Ist das erfindungsgemäße Mittel zur Anwendung in der von R.L. Mills et al. [Nature Vol. 336 (1988) S. 787] vorgeschlagenen Variante der Strahlentherapie bestimmt, so muß sich das Zentralion von einem Mößbauer-Isotop wie z.B. ⁵⁷Fe oder ¹⁵¹Eu ableiten.

Bei der Applikation der erfindungsgemäßen Mittel können diese auch zusammen mit einem geeigneten Träger wie z.B. Serum oder physiologischer Kochsalzlösung und/oder zusammen mit einem Protein wie z.B. Human Serum Albumin verabreicht werden. Die Dosierung ist dabei abhängig von der Art der zellulären Störung und den Eigenschaften des zur Anwendung kommenden Metallkomplexes.

Die nachfolgenden Beispiele erläutern die Erfindung, ohne sie auf diese beschränken zu wollen.

### Beispiel 1

### a) 3,6,9-Triaza-3,6,9-tris-(tert.-butoxycarbonylmethyl)-4-(4-methoxybenzyl)-undecandisäure-di-tert.-butyldiester

1,56 g (2 mmol) 3,6,9-Triaza-3,6,9-tris-(tert.-butoxycarbonylmethyl)-4-(4-hydroxybenzyl)-undecandisäure-ditert.-butyldiester (Beispiel 9f der DOS 3 710 730) werden in Tetrahydrofuran bei 0 °C mit 66 mg (2 2 mmol) 80 % Natriumhydrid versetzt. Dazu werden 0,31 g (2,2 mmol) Jodmethan gegeben und 30 Minuten gerührt. Dann wird die Lösung mit Wasser versetzt, Tetrahydrofuran abdestilliert und die wäßrige Emulsion mit Diethylether extrahiert. Die organische Phase wird mit Wasser gewaschen, über Na₂SO₄ getrocknet und eingeengt.
Ausbeute: 1,55 g (97,6 %)

| | | | |
|---|---|---|---|
| Ber.: | C 63,53 | H 9,01 | N 5,29 |
| Gef.: | C 63,37 | H 8,96 | N 5,32 |

### b) 3,6,9-Triaza-3,6,9-tris-(carboxymethyl)-4-(4-methoxybenzyl)-undecandisäure

1,27 g (1,6 mmol) des in Beispiel 1a) beschriebenen tert.-Butylesters werden in 25 ml Trifluoressigsäure gelöst und 1 Stunde bei Raumtemperatur gerührt. Anschließend wird die Lösung mit Diethylether versetzt, der Niederschlag abgesaugt, mit Ether gewaschen und bei 40 °C im Vakuum über Phosphorpentoxid getrocknet. Das Rohprodukt wird in Wasser gelöst und mit Aktivkohle verrührt. Man filtriert von der Kohle ab und lyophilisiert dreimal zur Entfernung von restlicher Trifluoressigsäure.
Ausbeute: 0,62 g (75,4 %)

| | | | |
|---|---|---|---|
| Ber.: | C 51,46 | H 6,09 | N 8,18 |
| Gef.: | C 51,27 | H 6,02 | N 8,11 |

### c) Gadoliniumkomplex der 3,6,9-Triaza-3,6,9-tris-(carboxymethyl)-4-(4-methoxybenzyl)-undecandisäure

513 mg ( 1 mmol) der in Beispiel 1b) beschriebenen Komplexbildnersäure werden in ca. 30 ml Wasser gelöst und bei 80 °C mit 181 mg (0,5 mmol) Gd₂O₃ versetzt. Nach 30 Minuten wird die fast klare Lösung filtriert und das Filtrat gefriergetrocknet.
Ausbeute: 649 mg (97,2 %), bezogen auf die wasserfreie Substanz.

| | | | | |
|---|---|---|---|---|
| Ber.: | C 39,57 | H 4,23 | N 6,29 | Gd 23,55 |
| Gef.: | C 39,47 | H 4,29 | N 6,21 | Gd 23,19 |

### Di-Natriumsalz des Gadoliniumkomplexes

Der wie vorstehend beschrieben erhaltene Komplex (500 mg, 0,75 mmol) wird in der 10fachen Menge Wasser gelöst und mittels einer Mikrobürette mit 1,5 ml einer 1 N Natronlauge versetzt.
Nach Gefriertrocknung liegen 533 mg weißer Kristalle vor.

Die T₁-Relaxivität (l/mmol.sec) beträgt
in Wasser 4,54 ± 0,13
in Plasma 6,89 ± 0,17

### Di-N-Methyl-D-Glucaminsalz des Gadoliniumkomplexes

3,34 g (5 mmol) des Gadoliniumkomplexes werden in 40 ml Wasser mit 1,96 g (10 mmol) N-Methyl-D-Glucamin unter Rühren portionsweise versetzt. Nach vollständigem Auflösen der Base wird gefriergetrocknet. Es bleiben 5,55 g einer farblosen kristallinen Verbindung zurück.

H₂O-Gehalt (Karl-Fischer-Bestimmung): 4,73 %

### d) Europiumkomplex der 3,6,9-Triaza-3,6,9-tris-(carboxymethyl)-4-(4-methoxybenzyl)-undecandisäure

5,13 g (10 mmol) der in Beispiel 1b beschriebenen Komplexbildnersäure werden in ca. 30 ml Wasser gelöst und bei 80°C mit 1,76 g (5 mmol) Eu₂O₃ versetzt. Nach 30 Minuten wird die fast klare Lösung filtriert und das Filtrat gefriergetrocknet.
Ausbeute: 6,62 g

| Analyse (bezogen auf wasserfreie Substanz) | | | | |
|---|---|---|---|---|
| ber.: | C 39,89 | H 4,26 | N 6,34 | Eu 22,94 |
| gef.: | C 39,71 | H 4,38 | N 6,17 | Eu 22,58 |

### Di-Natriumsalz des Europiumkomplexes

Der wie vorstehend beschriebene Komplex (497 mg, 0,75 mmol) wird in der 10fachen Menge Wasser gelöst und mittels einer Mikrobürette mit 1,5 ml einer 1N Natronlauge versetzt. Nach Gefriertrocknung liegen 540 mg weißer Kristalle vor.

### Di-N-Methyl-D-Glucaminsalz des Europiumkomplexes

3,31 g (5 mmol) des Europiumkomplexes werden in 40 ml Wasser mit 1,96 g (10 mmol) N-Methyl-D-Glucamin unter Rühren portionsweise versetzt. Nach vollständigem Auflösen der Base wird gefriergetrocknet. Es bleiben 5,63 g einer farblosen kristallinen Verbindung zurück.

### e) Eisen-III-komplex der 3,6,9-Triaza-3,6,9-tris-(carboxymethyl)-4-(4-methoxybenzyl)-undecandisäure

5,13 g (10 mmol) der in Beispiel 1b beschriebenen Komplexbildnersäure werden in ca. 30 ml Wasser gelöst und bei 80°C mit 798 mg (5 mmol) Fe₂O₃ versetzt. Nach 30 Minuten wird die fast klare Lösung filtriert und das Filtrat gefriergetrocknet. Ausbeute: 5,66 g

| Analyse (bezogen auf wasserfreie Substanz) : | | | | |
|---|---|---|---|---|
| ber.: | C 46,66 | H 4,98 | N 7,42 | Fe 9,86 |
| gef.: | C 46,71 | H 5,03 | N 7,38 | Fe 9,81 |

### Di-Natriumsalz des Eisen-III-komplexes

Der wie vorstehend beschrieben erhaltene Komplex (425 mg, 0,75 mmol) wird in der 10fachen Menge Wasser gelöst und mittels einer Mikrobürette mit 1,5 ml einer 1N Natronlauge versetzt. Nach Gefriertrocknung liegen 460 mg weißer Kristalle vor.

### Di-N-Methyl-D-Glucaminsalz des Eisen-III-komplexes

2,83 g (5 mmol) des Eisen-III-komplexes werden in 40 ml Wasser mit 1,96 g (10 mmol) N-Methyl-D-Glucamin unter Rühren portionsweise versetzt. Nach vollständigem Auflösen der Base wird gefriergetrocknet. Es bleiben 4,83 g einer farblosen kristallinen Verbindung zurück.

In analoger Weise erhält man mit Wismutoxid, Bi₂O₃, den Wismutkomplex als Di-Natriumsalz bzw. als Di-N-Methyl-D-Glucaminsalz.

### Beispiel 2

### a) 3,6,9-Triaza-3,6,9-tris-(tert.-butoxycarbonylmethyl)-5-(4-methoxybenzyl)-undecandisäure-di-tert.-butylester

3,9 g (5 mmol) 3,6,9-Triaza-3,6,9-tris-(tert.-butoxycarbonylmethyl)-5-(4-hydroxybenzyl)-undecandisäure-di-tert.-butylester (Beispiel 17d der DOS 3 710 730) werden nach der in Beispiel 1a) gegebenen Vorschrift zu 3,61 g (91 % der Theorie) der Titelverbindung umgesetzt.

| | | | |
|---|---|---|---|
| Ber.: | C 63,53 | H 9,01 | N 5,29 |
| Gef.: | C 63,59 | H 9,07 | N 5,27 |

### b) 3,6,9-Triaza-3,6,9-tris-(carboxymethyl)-5-(4-methoxybenzyl)-undecandisäure

3,18 g (4 mmol) des in Beispiel 2a) beschriebenen tert.-Butylesters werden nach der in Beispiel 1b) gegebenen Vorschrift mit Trifluoressigsäure behandelt und aufgearbeitet. Man erhält 1,62 g (79 % der Theorie) farbloses Lyophilisat.

| | | | |
|---|---|---|---|
| Ber.: | C 51,46 | H 6,09 | N 8,18 |
| Gef.: | C 51.34 | H 6,14 | N 8,11 |

### c) Gadoliniumkomplex der 3,6,9-Triaza-3,6,9-tris-(carboxymethyl)-5-(4-methoxybenzyl)-undecandisäure

1,03 g (2 mmol) der in Beispiel 2b) beschriebenen Komplexbildnersäure werden nach der Vorschrift in Beispiel 1c) mit Gd₂O₃ komplexiert. Man erhält 1,32 g (99 % der Theorie) farbloses Lyophilisat.

| | | | | |
|---|---|---|---|---|
| Ber.: | C 39,57 | H 4,23 | N 6,29 | Gd 23,55 |
| Gef.: | C 39,51 | H 4,19 | N 6,25 | Gd 23,61 |

Die T₁-Relaxivität (l/mmol.sec) beträgt
in Wasser 4,17 ± 0,14
in Plasma 6,61 ± 0,18

### Beispiel 3

### a) 3,6,9-Triaza-3,6,9-tris-(tert.-butoxycarbonylmethyl)-4-[4-(4-methoxybenzyloxy)-benzyl]-undecandisäure-di-tert.-butylester

1,56 g (2 mmol) 3,6,9-Triaza-3,6,9-tris-(tert.-butoxycarbonylmethyl)-4-(4-hydroxybenzyl)-undecandisäure-di-tert-butylester (Beispiel 9f der DOS 3 710 730) werden in Tetrahydrofuran bei 0 °C mit 66 mg (2,2 mmol) 80 % Natriumhydrid versetzt. Dazu werden 0,3 ml (2,2 mmol) 4-Methoxybenzylchlorid gegeben und über Nacht gerührt. Dann wird die Lösung mit Wasser versetzt, Tetrahydrofuran abdestilliert und die wäßrige Emulsion mit Diethylether extrahiert. Die organische Phase wird mit Wasser gewaschen, über Na₂SO₄ getrocknet und eingeengt. Das erhaltene farblose Öl wird an Kieselgel (Ether/Hexan 1:1) chromatographiert. Ausbeute: 1,17 g (65 % der Theorie) farbloses Öl.

| | | | |
|---|---|---|---|
| Ber.: | C 65,38 | H 8,62 | N 4,67 |
| Gef.: | C 65,29 | H 8,65 | N 4,59 |

### b) 3,6,9-Triaza-3,6,9-tris-(carboxymethyl)-4-[4-(4-methoxybenzyloxy)-benzyl]-undecandisäure

1,80 g (2 mmol) des in Beispiel 3a) beschriebenen tert.-Butylesters werden analog der für Beispiel 1b) gegebenen Vorschrift mit Trifluoressigsäure behandelt und zu 905 mg (73 % der Theorie) farblosem, flockigem Lyophilisat umgesetzt.

| | | | |
|---|---|---|---|
| Ber.: | C 56,21 | H 6,02 | N 6,78 |
| Gef.: | C 56,10 | H 5,98 | N 6,82 |

### c) Gadoliniumkomplex der 3,6,9-Triaza-3,6,9-tris-(carboxymethyl)-4-[4-(4-methoxybenzyloxy) -benzyl]-undecandisäure

620 mg (1 mmol) der in Beispiel 3b) beschriebenen Komplexbildnersäure werden analog der für Beispiel 1c) gegebenen Vorschrift komplexiert und aufgearbeitet. Man erhält 758 mg (98 % der Theorie).

| | | | | |
|---|---|---|---|---|
| Ber.: | C 45,01 | H 4,43 | N 5,43 | Gd 20,32 |
| Gef.: | C 44,93 | H 4,49 | N 5,37 | Gd 20,18 |

Die T₁-Relaxivität (l/mmol.sec) beträgt
in Wasser 4,23 ± 0,16
in Plasma 6,99 ± 0,13

### Beispiel 4

### a) Diethylphosphat des 3,6,9-Triaza-3,6,9-tris-(tert.-butoxycarbonylmethyl)-4-(4-hydroxybenzyl)-undecandisäure-di-tert.-butylesters

11,2 g (14,36 mmol) des in DOS 3 710 730 (Beispiel 9f) beschriebenen Phenols werden in 100 ml absolutem Tetrahydrofuran (THF) gelöst. Dazu werden 380 mg (15,8 mmol) Natriumhydrid gegeben (hergestellt aus 50 % NaH in Paraffinöl durch dreimaliges Waschen mit 10 ml THF). Nach 30 Minuten bei Raumtemperatur werden 2,60 g (15,0 mmol) Phosphorsäurediethylesterchlorid zugesetzt und 24 Stunden bei Raumtemperatur gerührt.

Die Lösung wird mit 500 ml Ether verdünnt und dreimal mit 300 ml 10 % Natronlauge gewaschen. Nach Trocknen der organischen Phase über Magnesiumsulfat wird im Vakuum eingeengt und der Rückstand durch Flash-Chromatographie (Laufmittel: Ether/Hexan = 1:1) aufgereinigt. Ausbeute: 11,97 g (91 % der Theorie) eines blaßgelben Öls.

| | | | | |
|---|---|---|---|---|
| Ber.: | C 59,00 | H 8,58 | N 4,59 | P 3,38 |
| Gef.: | C 58,88 | H 8,63 | N 4,63 | P 3,30 |

### b) 3,6,9-Triaza-3,6,9-tris-(tert-butoxycarbonylmethyl)-4-benzyl-undecandisäure-di-tert.-butylester

Eine Mischung aus 1,33 g (8,62 mmol) wasserfreiem Titan(III)-chlorid und 1,02 g (26,09 mmol) feingeschnittenem Kalium in 20 ml Tetrahydrofuran wird in einer Argonatmosphäre 1 Stunde unter Rückfluß erhitzt.

Zu dieser Mischung wird innerhalb 15 Minuten eine Lösung von 11,5 g (12,55 mmol) der in Beispiel 4a) beschriebenen Verbindung in 50 ml Tetrahydrofuran zugetropft. Anschließend wird 8 Stunden unter Rückfluß erhitzt. Man kühlt im Eisbad, gibt vorsichtig 20 ml Methanol, dann 100 ml Wasser zu und extrahiert dreimal mit 200 ml Ether. Die organischen Phasen werden über Magnesiumsulfat getrocknet und im Vakuum eingeengt. Der Rückstand wird an Kieselgel chromatographiert (Laufmittel: Hexan/Ether = 2:1). Man erhält 8,9 g (93 % der Theorie) der Titelverbindung als farbloses Öl, das beim Stehenlassen kristallisiert.

| | | | |
|---|---|---|---|
| Ber.: | C 64,46 | H 9,10 | N 5,50 |
| Gef.: | C 64,54 | H 9,15 | N 5,41 |

### c) 3,6,9-Triaza-3,6,9-tris-(carboxymethyl)-4-benzyl-undecandisäure

7,64 g (10 mmol) des in Beispiel 4b) beschriebenen tert.-Butylesters werden analog der für Beispiel 1b) gegebenen Vorschrift zu 4,01 (83 % der Theorie) der Titelverbindung umgesetzt.

| | | | |
|---|---|---|---|
| Ber.: | C 52,17 | H 6,05 | N 8,69 |
| Gef.: | C 52,23 | H 5,99 | N 8,73 |

### d) Gadoliniumkomplex der 3,6,9-Triaza-3,6,9-tris-(carboxymethyl)-4-benzyl-undecandisäure

2,42 g (5 mmol) der in Beispiel 4c) beschriebenen Komplexbildnersäure werden analog der in Beispiel 1c) gegebenen Vorschrift zu 3,14 g (98.5 % der Theorie) der Titelverbindung umgesetzt. Man erhält den Gadolinium-Komplex als farbloses, flockiges Lyophilisat.

| | | | | |
|---|---|---|---|---|
| Ber.: | C 39,55 | H 4,11 | N 6,59 | Gd 24,66 |
| Gef.: | C 39,47 | H 4,19 | N 6,52 | Gd 24,88 |

Die T₁-Relaxivität (l/mmol.sec) beträgt
in Wasser 4,54 ± 0,13
in Plasma 6,89 ± 0,17

### Ytterbiumkomplex der 3,6,9-Triaza-3,6,9-tris-(carboxymethyl)-4-benzyl-undecandisäure

Analog der Vorschrift zur Herstellung des Gadoliniumkomplexes erhält man, wenn man Yb₂O₃ anstelle von Gd₂O₃ einsetzt, den entsprechenden Ytterbiumkomplex.

### Beispiel 5

### a) 3,6,9-Triaza-3,6,9-tris-(tert.-butoxycarbonylmethyl)-4-benzyloxymethyl-undecandisäure-di-tert.-butylester

Zu einer gut gerührten Suspension aus 14,1 g (20 mmol) des in DOS 3 710 730 (Beispiel 37d) beschriebenen 4-Hydroxymethyl-3,6,9-triaza-3,6,9-tris-(tert.-butoxycarbonylmethyl)-undecan-disäure-tert.-butyldiesters und 0,3 g Tetrabutylammonium-hydrogensulfat in 200 ml Dichlormethan / 200 ml 30 %iger Natronlauge tropft man 7,2 ml (60 mmol) Benzylbromid innerhalb 30 Minuten bei Raumtemperatur und rührt anschließend 8 Stunden.

Zu dieser Suspension werden 400 ml Wasser gegeben, die organische Phase wird abgetrennt und die wäßrige Phase zweimal mit je 150 ml Dichlormethan extrahiert. Nach Trocknen der vereinigten organischen Phasen über Magnesiumsulfat wird an Kieselgel (Ether/Hexan = 1:1) chromatographiert. Man erhält 13,0 g (82 % der Theorie) der Titelverbindung als farbloses Öl.

| | | | |
|---|---|---|---|
| Ber.: | C 63,53 | H 9,01 | N 5,29 |
| Gef.: | C 63,42 | H 9,07 | N 5,21 |

### b) 3,6,9-Triaza-3,6,9-tris-(carboxmethyl)-4-benzyloxymethyl-undecandisäure

7,94 g (10 mmol) des in Beispiel 5a) beschriebenen tert.-Butylesters werden analog der für Beispiel 1b) gegebenen Vorschrift mit Trifluoressigsäure zu 4,06 g (79 % der Theorie) der Titelverbindung umgesetzt.

| | | | |
|---|---|---|---|
| Ber.: | C 51,46 | H 6,09 | N 8,18 |
| Gef.: | C 51.51 | H 6,06 | N 8,12 |

### c) Gadoliniumkomplex der 3,6,9-Triaza-3,6,9-tris-(carboxymethyl)-4-benzyloxymethyl-undecandisäure

2,57 g (5 mmol) der in Beispiel 5b) beschriebenen Komplexbildnersäure werden analog der für Beispiel 1c) gegebenen Vorschrift zu 3,30 g (98,9 % der Theorie) der Titelverbindung umgesetzt. Man bekommt einen farblosen, flockigen Feststoff.

| | | | | |
|---|---|---|---|---|
| Ber.: | C 39,57 | H 4,23 | N 6,29 | Gd 23,55 |
| Gef.: | C 39,51 | H 4,26 | N 6,35 | Gd 23,27 |

Die T₁-Relaxivität (l/mmol.sec) beträgt
in Wasser 4,39 ± 0,12
in Plasma 6,31 ± 0,15

### Beispiel 6

### a) 3,6,9-Triaza-3,6,9-tris-(tert.-butoxycarbonylmethyl)-4-(4-carboxymethoxybenzyl)-undecandisäure-bis-(tert-butyl)-ester

23,40 g (30 mmol) 3,6,9-Triaza-3,6,9-tris-(tert.-butoxycarbonylmethyl)-4-(4-hydroxybenzyl)-undecandisäure-di-tert-butylester (Beispiel 9f der DOS 3 710 730) werden in Tetrahydrofuran bei 0 °C mit 2,7 g (90 mmol) 80 %igem Natriumhydrid versetzt. Dazu werden 6,25 g (45 mmol) Bromessigsäure in Tetrahydrofuran zugetropft. 1 Stunde bei 0 °C und über Nacht bei Raumtemperatur gerührt.

Dann wird die Lösung mit Wasser versetzt, Tetrahydrofuran abdestilliert und die wäßrige Phase mit Essigsäureethylester extrahiert. Die organische Phase wird über Natriumsulfat getrocknet und eingeengt.

Der Rückstand wird im Laufmittelgemisch Dioxan/Methanol/Triethylamin (15:4:1) an Kieselgel chromatographiert; die vereinigten Fraktionen werden eingeengt und zwischen Essigsäureethylester und 1 N Zitronensäure verteilt. Die organische Phase wird dann über Natriumsulfat getrocknet und eingeengt. Man erhält 21,8 g (87 % der Theorie) als farbloses Öl.

| | | | |
|---|---|---|---|
| Ber.: | C 61,63 | H 8,54 | N 5,01 |
| Gef.: | C 61,62 | H 8,62 | N 4,95 |

### b) 3,6,9-Triaza-3,6,9-tris-(carboxymethyl)-4-(4-carboxymethoxybenzyl)-undecandisäure

21,0 g (25 mmol) des in Beispiel 6a) beschriebenen tert.-Butylesters werden analog der für Beispiel 1b) gegebenen Vorschrift zu 11,0 g (78,9 % der Theorie) Titelverbindung umgesetzt.

| | | | |
|---|---|---|---|
| Ber.: | C 49,55 | H 5,60 | N 7,54 |
| Gef.: | C 49,31 | H 5,51 | N 7,47 |

### c) Gadoliniumkomplex der 3,6,9-Triaza-3,6,9-tris-(carboxymethyl)-4-(4-carboxymethoxybenzyl)-undecandisäure

5.57 g (10 mmol) der in Beispiel 6b) beschriebenen Komplexbildnersäure werden analog der für Beispiel 1c) gegebenen Vorschrift zu 7,01 g (98,5 % der Theorie) der Titelverbindung umgesetzt.

| | | | | |
|---|---|---|---|---|
| Ber.: | C 38,81 | H 3,96 | N 5,90 | Gd 22.09 |
| Gef.: | C 38,75 | H 3,89 | N 5,97 | Gd 21,93 |

Die T₁-Relaxivität (l/mmol.sec) beträgt
in Wasser 5,00 ± 0,01
in Plasma 7,10 ± 0,08

### Beispiel 7

### Herstellung einer Lösung des Natriumsalzes des Gadolinium-III-Komplexes der 3,6,9-Triaza-3,6,9-tris-(carboxymethyl)-4-benzyloxymethyl-undecandisäure

6,68 g (10 mmol) des nach Beispiel 5c) erhaltenen Gadoliniumkomplexes werden in 70 ml Wasser pro injectione (p.i.) gelöst und tropfenweise mit 1 N Natronlauge versetzt, bis ein pH von 7,2 erreicht ist. Nach Zugabe von 0,02 g Tromethamin wird mit Wasser p.i. auf 100 ml aufgefüllt, die Lösung in Flaschen abgefüllt und hitzesterilisiert.

### Beispiel 8

### a) 3,6,9-Triaza-3,6,9-tris(tert.-butoxycarbonylmethyl)-4-(4-ethoxybenzyl)-undecandisäure-di-tert.-butyldiester

5,85 g (7,5 mmol) 3,6,9-Triaza-3,6,9-tris(tert.-butoxycarbonylmethyl)-4-(4-hydroxybenzyl)-undecandisäure-di-tert.-butyldiester (Beispiel 9f der DOS 3710 730) werden in 100 ml Tetrahydrofuran bei 0°C mit 0,30 g (10 mmol) 80 % Natriumhydrid versetzt. Dazu werden 1,56 g (10 mmol) Jodethan gegeben und 3 Stunden gerührt. Dann wird die Lösung mit Wasser versetzt, Tetrahydrofuran abdestilliert und die wässrige Emulsion mit Diethylether extrahiert. Das nach Trocknen über Natriumsulfat und Einengen des Lösungsmittels erhaltene Rohprodukt wird an Kieselgel (System: Hexan/Ether/Triethylamin 70:30:5) chromatographiert. Ausbeute: 4,0 g (66 %)

| Analyse (bezogen auf wasserfreie Substanz): | | | |
|---|---|---|---|
| ber.: | C 63,91 | H 9,11 | N 5,20 |
| gef.: | C 63,67 | H 9,05 | N 5,28 |

### b) 3,6,9-Triaza-3,6,9-tris(carboxymethyl)-4-(4-ethoxybenzyl)-undecandisäure

3,64 g (4,5 mmol) des in Beispiel 8a beschriebenen tert.-Butylesters werden in 25 ml Trifluoressigsäure gelöst, eine Stunde bei Raumtemperatur gerührt und analog Beispiel 1b aufgearbeitet. Ausbeute: 1,2 g (50,6 %)

| Analyse (bezogen auf wasserfreie Substanz): | | | |
|---|---|---|---|
| ber.: | C 52,36 | H 6,31 | N 7,97 |
| gef.: | C 52,21 | H 6,39 | N 7,84 |

### c) Di-Natriumsalz des Gadoliniumkomplexes der 3,6,9-Triaza-3,6,9-tris(carboxymethyl)-4-(4-ethoxybenzyl)-undecandisäure

528 mg (1 mmol) der im vorstehenden Beispiel beschriebenen Komplexbildnersäure werden in 40 ml Wasser gelöst und bei 80°C mit 181 mg (0,5 mmol) Gd₂O₃ komplexiert. Anschließend wird mit 2 ml 1N NaOH neutralisiert, mit Aktivkohle gerührt, filtriert und das Filtrat gefriergetrocknet. Ausbeute: 700 mg (96,5 %)

| Analyse (bezogen auf wasserfreie Substanz): | | | | | |
|---|---|---|---|---|---|
| ber.: | C 38,06 | H 3,89 | Gd 21,67 | N 5,79 | Na 6,34 |
| gef.: | C 37,91 | H 3,99 | Gd 21,30 | N 5,69 | Na 6,57 |

Die T₁-Relaxivität (l/mmol·sec) beträgt
in Wasser 5.33 ± 0,13
in Plasma 8,69 ± 0,53
In analoger Weise erhält man mit Europiumoxid, Eu₂O₃ den entsprechenden Europiumkomplex:

| | | | | | |
|---|---|---|---|---|---|
| ber.: | C 38,34 | H 3,92 | Eu 21,09 | N 5,83 | Na 6,38 |
| gef.: | C 38,20 | H 4,01 | Eu 20,87 | N 5,79 | Na 6,49 |

In analoger Weise erhält man mit Eisenoxid, Fe₂O₃, den entsprechenden Eisenkomplex:

| | | | | | |
|---|---|---|---|---|---|
| ber.: | C 44,25 | H 4,52 | Fe 8,95 | N 6,73 | Na 7,37 |
| gef.: | C 44,17 | H 4,59 | Fe 8,52 | N 6,81 | Na 7,49 |

### Beispiel 9

### a) 3,6,9-Triaza-3,6,9-tris(tert.-butoxycarbonylmethyl)-4-(4-butoxybenzyl-undecandisäure-di-tert.-butyldiester

5,85 g (7,5 mmol) 3,6,9-Triaza-3,6,9-tris(tert.-butoxycarbonylmethyl)-4-(4-hydroxybenzyl)-undecandisäure-di-tert.-butyldiester (Beispiel 9f der DOS 3710 730) werden analog Beispiel 8a mit 1,84 g (10 mmol) 1-Jodbutan umgesetzt und wie dort beschrieben, aufgearbeitet. Ausbeute: 4,1 g (65,4 %)

| Analyse (bezogen auf wasserfreie Substanz): | | | |
|---|---|---|---|
| ber.: | C 64,64 | H 9,28 | N 5,03 |
| gef.: | C 64,82 | H 9,37 | N 4,96 |

### b) 3,6,9-Triaza-3,6,9-tris(carboxymethyl)-4-(4-butoxybenzyl)-undecandisäure

3,34 g (4 mmol) des in Beispiel 9a beschriebenen tert.-Butylesters werden in 20 ml Trifluoressigsäure gelöst, eine Stunde bei Raumtemperatur gerührt und analog zu Beispiel 1b aufgearbeitet. Ausbeute: 1,36 g (61,0 %)

| Analyse (bezogen auf wasserfreie Substanz): | | | |
|---|---|---|---|
| ber.: | C 54,04 | H 6,71 | N 7,57 |
| gef.: | C 53,88 | H 6,63 | N 7,41 |

### c) Di-Natriumsalz des Gadoliniumkomplexes der 3,6,9-Triaza-3,6,9-tris(carboxymethyl)-4-(4-butoxybenzyl)-undecandisäure

556 mg (1 mmol) der im vorstehenden Beispiel beschriebenen Komplexbildnersäure werden mit 40 ml Wasser versetzt und bei 80°C mit 181 mg (0,5 mmol) Gd₂O₃ komplexiert. Anschließend wird mit 2 ml 1N NaOH neutralisiert, mit Aktivkohle gerührt, filtriert und das Filtrat gefriergetrocknet. Ausbeute: 711 mg (94,3 %)

| Analyse (bezogen auf wasserfreie Substanz): | | | | | |
|---|---|---|---|---|---|
| ber.: | C 39,83 | H 4,28 | Gd 20,86 | N 5,58 | Na 6,10 |
| gef.: | C 39,61 | H 4,35 | Gd 20,51 | N 5,49 | Na 6,17 |

Die T₁-Relaxivität (l/mmol·s) beträgt
in Wasser 5,80 ± 0,26
in Plasma 14,20 ± 0,98

In analoger Weise erhält man mit Europiumoxid, Eu₂O₃, den entsprechenden Europiumkomplex:

| | | | | | |
|---|---|---|---|---|---|
| ber.: | C 40,11 | H 4,31 | Eu 20,30 | N 5,61 | Na 6,14 |
| gef.: | C 39,97 | H 4,39 | Eu 20,02 | N 5,72 | Na 6,25 |

In analoger Weise erhält man mit Eisenoxid, Fe₂O₃, den entsprechenden Eisenkomplex:

| | | | | | |
|---|---|---|---|---|---|
| ber.: | C 46,03 | H 4,94 | Fe 8,56 | N 6,44 | Na 7,05 |
| gef.: | C 45,88 | H 5,03 | Fe 8,30 | N 6,50 | Na 7,11 |

### Beispiel 10

### a) 3,6,9-Triaza-3,6,9-tris(tert.-butoxycarbonylmethyl)-4-(4-benzyloxybenzyl)-undecandisäure-di-tert.-butyldiester

5,85 g (7,5 mmol) 3,6,9-Triaza-3,6,9-tris(tert.-butoxycarbonylmethyl-4-(4-hydroxybenzyl)-undecandisäure-di-tert.-butyldiester (Beispiel 9f der DOS 3710 730) werden analog Beispiel 8a mit 1,71 g (10 mmmol) Benzylbromid umgesetzt und wie dort beschrieben, aufgearbeitet.
Ausbeute: 4,9 g (75,1 %)

| Analyse (bezogen auf wasserfreie Substanz). | | | |
|---|---|---|---|
| ber.: | C 66,25 | H 8,69 | N 4,83 |
| gef.: | C 66,14 | H 8,77 | N 4,83 |

### b) 3,6,9-Triaza-3,6,9-tris(carboxymethyl)-4-(4-benzyloxybenzyl)-undecandisäure

3,48 g (4 mmol) des in Beispiel 10a beschriebenen tert.-Butylesters werden in 20 ml Trifluoressigsäure gelöst, eine Stunde bei Raumtemperatur gerührt und analog Beispiel 1b aufgearbeitet.
Ausbeute: 1,33 g (56,5 %)

| Analyse (bezogen auf wasserfreie Substanz): | | | |
|---|---|---|---|
| ber.: | C 57,04 | H 5,98 | N 7,13 |
| gef.: | C 56,89 | H 6,03 | N 7,21 |

### c) Di-Natriumsalz des Gadoliniumkomplexes der 3,6,9-Triaza-3,6,9-tris(carboxymethyl)-4-(4-benzyloxybenzyl)-undecandisäure

590 mg (1 mmol) der im vorstehenden Beispiel beschriebenen Komplexbildnersäure werden mit 40 ml Wasser und 1 ml 1N NaOH versetzt und bei 80°C mit 181 mg (0,5 mmol) Gd₂O₃ komplexiert. Anschließend wird noch mit 1 ml 1N NaOH neutralisiert, mit Aktivkohle gerührt, filtriert und das Filtrat gefriergetrocknet.
Ausbeute: 703 mg (89,2 %)

| Analyse (bezogen auf wasserfreie Substanz): | | | | | |
|---|---|---|---|---|---|
| ber.: | C 42,69 | H 3,84 | Gd 19,96 | N 5,33 | Na 5,84 |
| gef.: | C 42,63 | H 3,91 | Gd 19,57 | N 5,26 | Na 5,99 |

Die T₁-Relaxivität (l/mmol·sec) beträgt
in Wasser 5,81 ± 0,11
in Plasma 16,35 ± 1,01
In analoger Weise erhält man mit Europiumoxid, Eu₂O₃, den entsprechenden Europiumkomplex:

| | | | | | |
|---|---|---|---|---|---|
| ber.: | C 42,98 | H 3,86 | Eu 19,42 | N 5,37 | Na 5,88 |
| gef.: | C 43,10 | H 3,91 | Eu 19,13 | N 5,27 | Na 5,99 |

In analoger Weise erhält man mit Eisenoxid, Fe₂O₃, den entsprechenden Eisenkomplex:

| | | | | | |
|---|---|---|---|---|---|
| ber.: | C 48,99 | H 4,41 | Fe 8,14 | N 6,12 | Na 6,70 |
| gef.: | C 48,73 | H 4,57 | Fe 8,29 | N 6,03 | Na 6,85 |

### Beispiele für eine in-vivo-NMR-Diagnostik

### Beispiel 1

Mit Hilfe eines Kernspintomographen der Firma General Electric, der speziell für die tierexperimentelle Forschung entwickelt worden ist, wurden Aufnahmen zu verschiedenen Zeiten nach Applikation des Di-Natriumsalzes des Gadolinium-Komplexes von Beispiel 1c) bei Ratten durchgeführt.

Mit dem Kernspintomographen (CSI 2 T) wurden Spin-Echo-Aufnahmen bei 2 Tesla (TR-Zeit von 400 ms und einer TE-Zeit von 20 ms) durchgeführt. Die Schichtdicke dieser T₁-gewichteten Aufnahmesequenz betrug 3 mm, die Aufnahmematrix war 128 x 128.

Das Kontrastmittel wurde intravenös in eine Schwanzvene einer männlichen Nacktratte (Lew/Mol), Gewicht 190 g, in einer Dosis von 0,06 mmol/kg appliziert. Das Tier hatte einen Brown-Pearce Tumor im Oberschenkel und wurde zur Untersuchung durch eine im. Gabe von Ketavet/Rompun narkotisiert.

In der coronaren Leeraufnahme (baseline, Nr. 1) sind verschiedene dunkle Strukturen in Abdomen sichtbar. Eine Differenzierung zwischen Darmlumen und Magen war nicht möglich.

Eine Minute nach Applikation (Nr. 2) ist bereits das erste Enhancement in der Harnblase sichtbar. Eine starke Kontrastzunahme ist im Magen 45 Minuten p.i. sichtbar (Nr. 3). 60 Minuten p.i. (Nr. 4) ist eine gute Darstellung des Tumors (in der Höhe des Referenzröhrchens) , der Harnblase und des Magens zu beobachten. Darüber hinaus ist ebenfalls eine Kontrastierung des Darms erkennbar. Dadurch ist eine Differenzierung von Darmschlingen, Fett sowie Lymphknoten (Lymphomen) möglich. Auffällig ist auch die Kontrastierung des Nierenbeckens. das 65 Minuten p.i. in einer etwas anderen Schicht noch besser dargestellt werden kann (Nr. 5). In der Abbildung Nr. 6, 180 Minuten p.i. ist das Kontrastenhancement auch in einer axialen Aufnahme im Bereich der Leber deutlich sichtbar. Hierdurch gelingt eine Differenzierung von Magen, Leber, Duodenum und Pankreas.

### Beispiel 2

Bei den Versuchstieren handelte es sich um weibliche Ratten des Stammes Lew/Mol mit einem Gewicht von 160 - 180 g. Vor dem Imaging wurden die Tiere narkotisiert (Rompun^{(R)} + Ketavet ^{(R)}) und für die Applikation des Kontrastmittels mit einem Katheter in der Schwanzvene versehen. Das Imaging erfolgte in einem MRI-Experimentalgerät der Firma General Electric (Feldstärke 2 Tesla). Zunächst wurden die Bilder (7, 9, 11) ohne Kontrastmittel mit einer T₁-gewichteten Spin-Echo Sequenz erstellt (TR = 400 msec, TE = 20 msec, aciale Schnittebene. Schichtdicke 3 mm). Die Leber erscheint jeweils mit der normalen Signalintensität; der Magen ist in der Tendenz dunkler als die Leber. Im Falle von Tier 1 weist der Magen zum Teil eine recht hohe Signalintensität auf. Dies ist auf Reste von Futter zurückzuführen, welches in relativ hohen Konzentrationen Mangan enthält (die Tiere hatten zum Zeitpunkt der Untersuchung 6 Stunden kein Futter erhalten). Tier 3 wurde 3 Wochen zuvor ein osteogenes Sarkom implantiert; dieses ist auf der Leeraufnahme isodens und nicht abgrenzbar. Die Kontrastmittelgabe erfolgte durch den Venenkatheter mit einer Dosis von 0,1 mmol Gd/kg (Konz. der Lösungen 0,05 mmol Gd/ml in 0,9 % NaCl) für alle 3 Substanzen.

Für alle 3 Substanzen ist nach 90 Minuten [Abb. 8. Beispiel 8c)] bzw. 60 Minuten p.a. [Abb. 10, Beispiel 9c)]; Abb. 12, Beispiel 10c)] ein deutliches Enhancement der Leber zu verzeichnen, welches auf die Aufnahme durch die Hepatozyten zurückzuführen ist und zu diesem Zeitpunkt nach Applikation bei dem bisher einzigen auf dem Markt befindlichen Kontrastmittel für die Kernspintomographie, Magnevist^{(R)}, nicht zu beobachten ist. Im Falle von Tier 3 [Abb. 12, Beispiel 10c)] erkennt man jetzt außerdem deutlich den Tumor, welcher das Kontrastmittel nicht bzw. zu einem geringeren Anteil aufgenommen hat.

Desweiteren zeigt sich bei allen Substanzen - am stärksten bei Beispiel 10c), am geringsten bei Beispiel 8c) - ein starkes Enhancement des Magens. Dies bietet zusätzliche diagnostische Möglichkeiten im Hinblick auf eine bessere Abgrenzung von Leber und Magen. 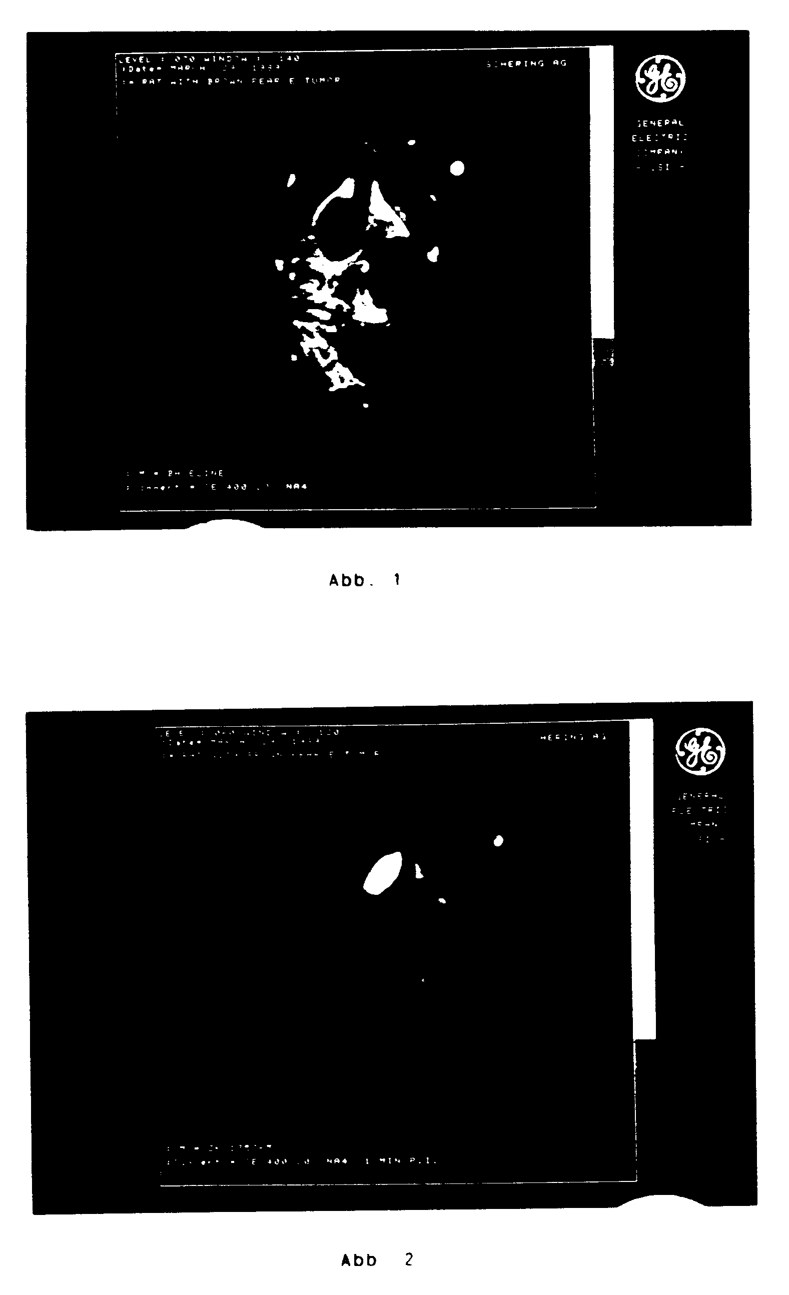 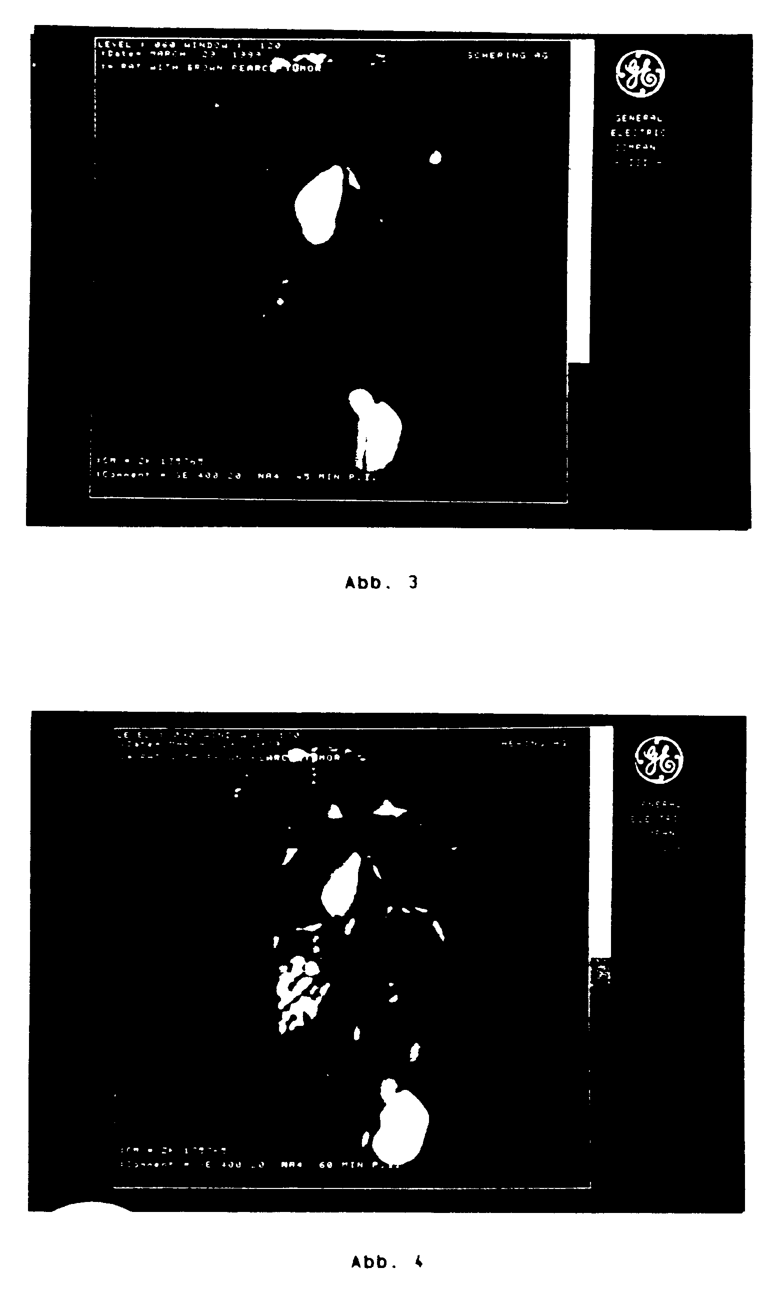 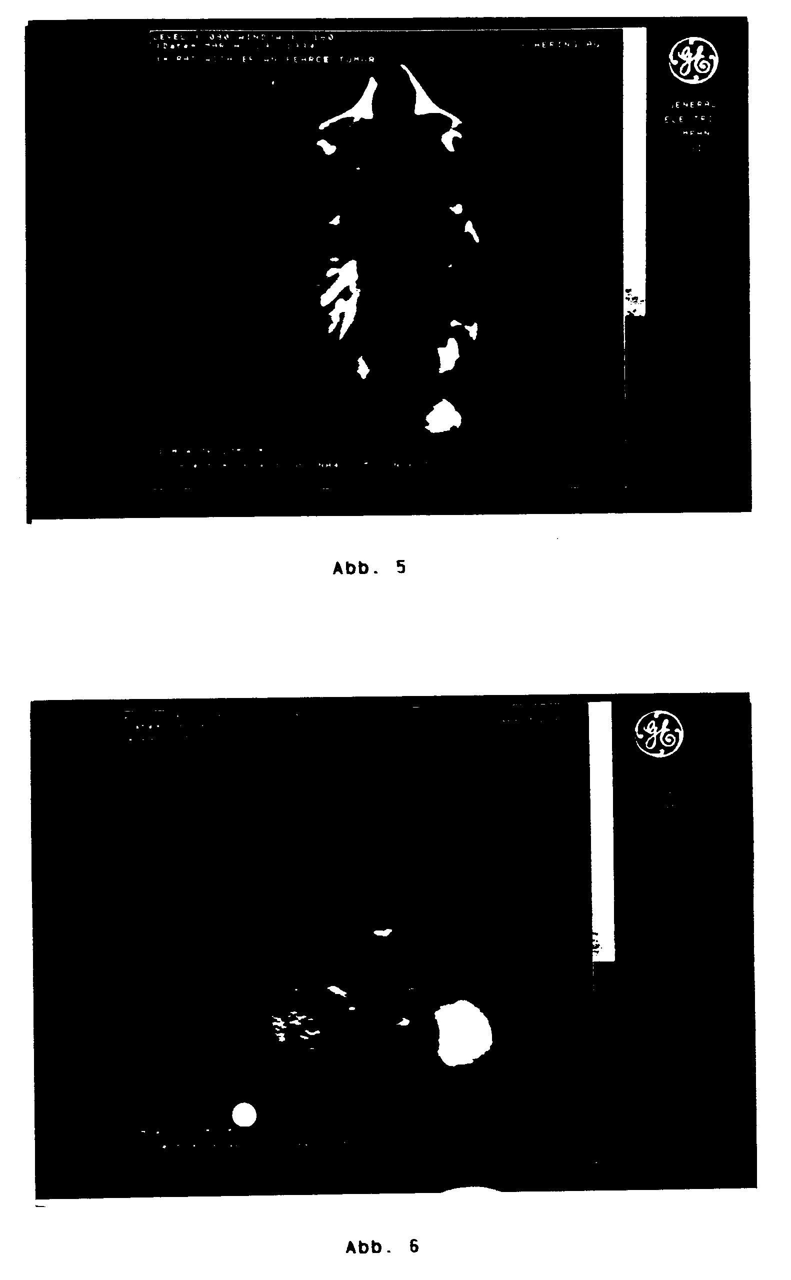 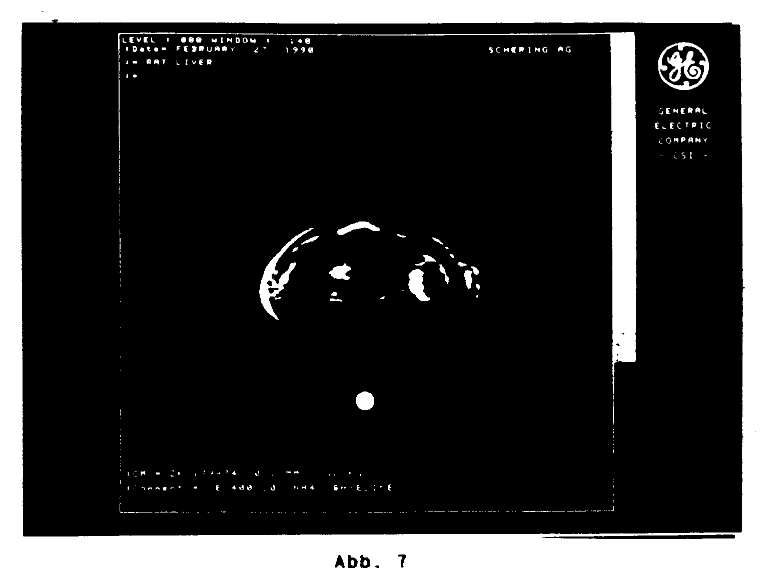
Axiales Bild von Rattenleber ohne Kontrastmittel (Spin-Echo-Sequenz TR=400 msec TE 20 msec Schichtdicke 3 mm) 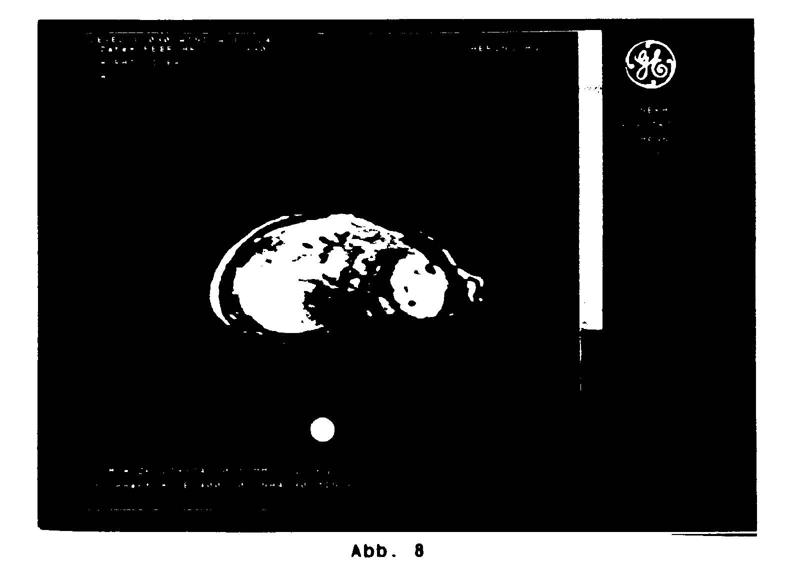
Axiales Bild von Rattenleber 90 Minuten nach Kontrastmittelgabe (Dosis 0,1 mmol Gd/ kg, gleiche Schnittebene und Aufnahmebedingungen wie bei Abb. 7) 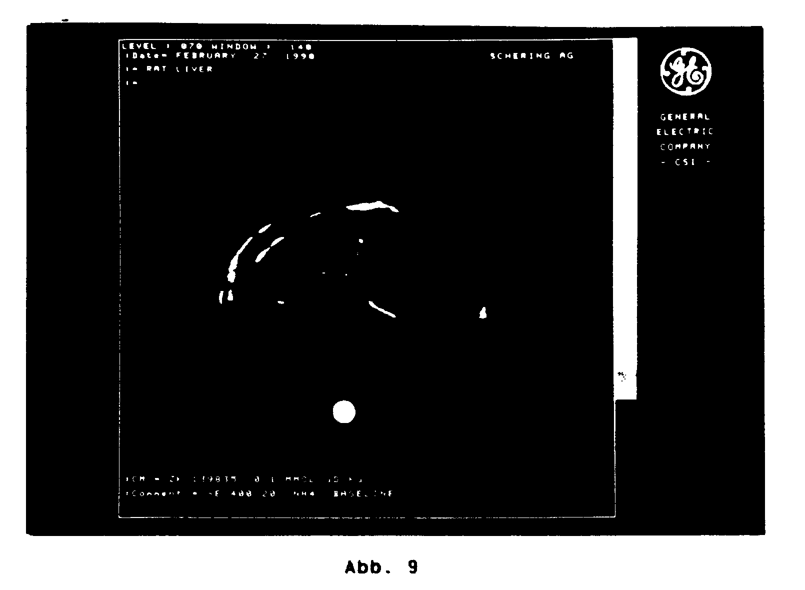
Axiales Bild von Rattenleber ohne Kontrastmittel (Spin-Echo-Sequenz TR=400 msec TE 20 msec. Schichtdicke 3 mm) 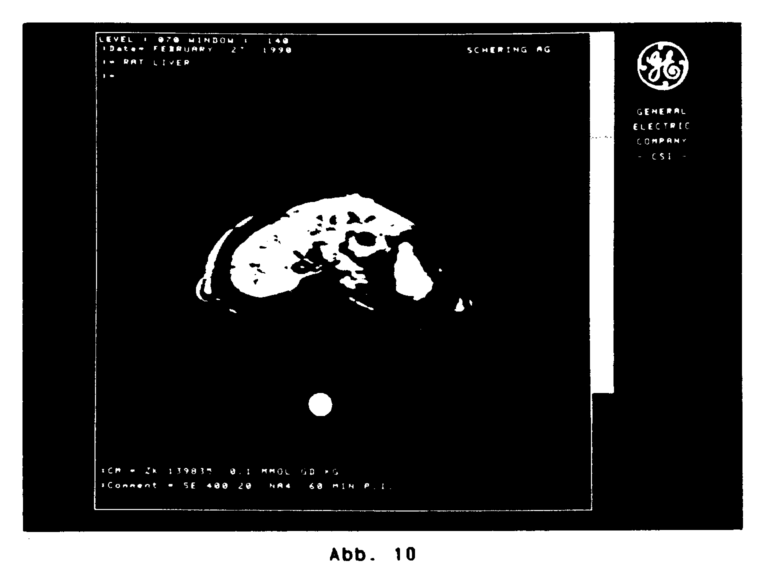
Axiales Bild von Rattenleber 60 Minuten nach Kontrastmittelgabe (Dosis 0,1 mmol Gd/kg, gleiche Schnittebene und Aufnahmebedingungen wie bei Abb. 9) 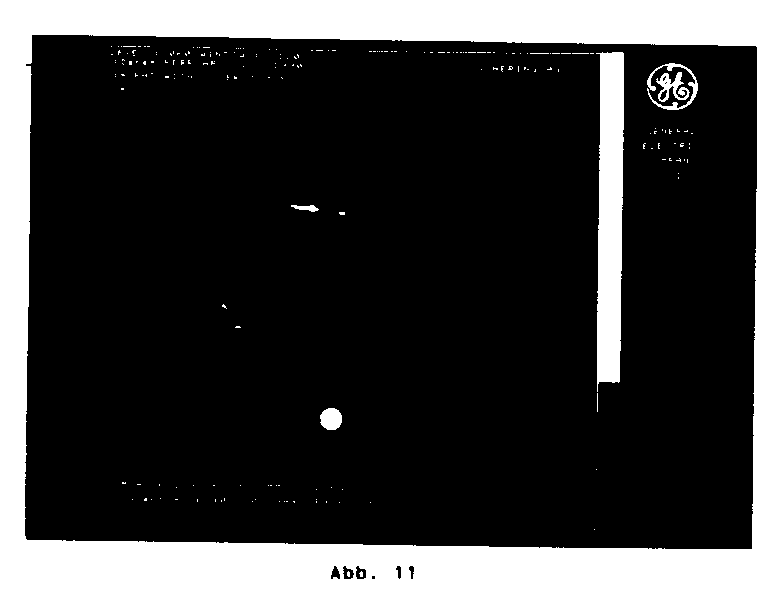
Axiales Bild von Rattenleber ohne Kontrastmittel (Spin-Echo-Sequenz TR=400 msec TE 20 msec Schichtdicke 3 mm) 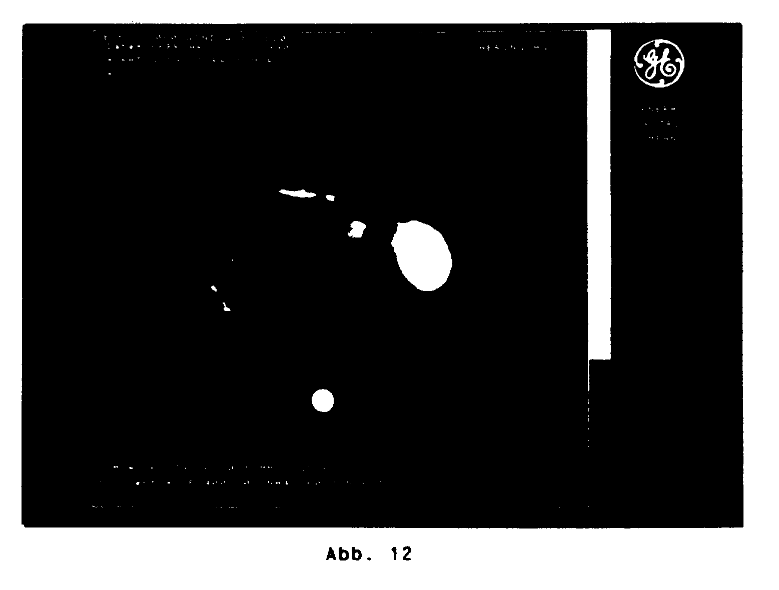
Axiales Bild von Rattenleber 60 Minuten nach Kontrastmittelgabe (Dosis 0. 1 mmol Gd/kg, gleiche Schnittebene und Aufnahmebedingungen wie bei Abb. 11). Im linken oberen Quadranten ist jetzt der Tumor deutlich abgrenzbar.

## Patentansprüche

1. Verbindungen der allgemeinen Formel I 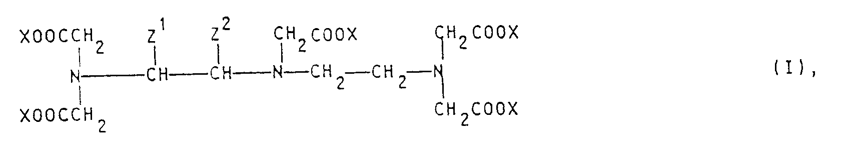 worin
Z¹ und Z² unabhängig voneinander für ein Wasserstoffatom oder den Rest -(CH₂)ₘ-(C₆H₄)_{q}-(O)ₖ-(CH₂)ₙ-(C₆H₄)ₗ-(O)ᵣ-R,
worin
m und n die Ziffern 0 - 20.
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms. eines C₁-C₆-Alkylrestes oder einer Benzylgruppe bedeuten.
X für ein Wasserstoffatom und/oder ein Metallionenäquivalent eines Elements der Ordnungszahlen 21 - 29, 42, 44 oder 57 - 83 stehen, mit der Maßgabe, daß mindestens zwei der Substituenten X für ein Metallionenäquivalent stehen, daß einer der Substituenten Z¹ und Z² für ein Wasserstoffatom und der andere nicht für ein Wasserstoffatom steht, daß - wenn n und l jeweils für die Ziffer 0 stehen -
k und r nicht gleichzeitigjeweils die Ziffer 1 bedeuten, daß Z¹ oder Z² nicht für -CH₂-C₆H₄-O-CH₂-COOCH₂C₆H₅ oder -CH₂-C₆H₄-O-(CH₂)₅-COOCH₂C₆H₅ sieben, daß -(O)ᵣ-R nicht für -OH steht, daß Z¹ nicht für 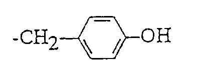 steht und daß die Summe von q und l die Ziffer 1 oder 2 ergibt, sowie deren Salze mit anorganischen und/oder organischen Basen, Aminosäuren oder Aminosäureamiden.

2. Verbindungen gemäß Anspruch 1, dadurch gekennzeichnet, daß Z¹ für ein Wasserstoffatom und Z² für den Rest - (CH₂)ₘ-(C₆H₄)_{q}-(O)ₖ-(CH₂)ₙ-(C₆H₄)ₗ-(O)ᵣ-R steht.

3. Verbindungen gemäß Anspruch 1, dadurch gekennzeichnet, daß Z² für ein Wasserstoffatom und Z¹ für den Rest -(CH₂)ₘ-(C₆H₄)_{q}-(O)ₖ-(CH₂)ₙ-(C₆H₄)ₗ-(O)ᵣ-R steht.

4. Verbindungen gemaß Anspruch 1, dadurch gekennzeichnet, daß Z¹ bzw. Z² für die Reste -CH₂-C₆H₄-OCH₃, -CH₂-C₆H₅, -CH₂-C₆H₄-O-CH₂-C₆H₄-OCH₃, -CH₂-O-CH₂-C₆H₅, -CH₂-C₆H₄-O-CH₂-COOH, -CH₂-C₆H₄-OC₂H₅, -CH₂-C₆H₄-OC₄H₉, -CH₂-C₆H₄-O-CH₂-C₆H₅ steht.

5. Die Verbindungen gemäß Anspruch 1:
Gadoliniumkomplex der 3,6,9-Triaza-3,6,9-tris(carboxymethyl)-4-(4-methoxybenzyl)-undecandisäure;
Europiumkomplex der 3,6,9-Triaza-3,6,9-tris(carboxymethyl)-4-(4-methoxybenzyl)-undecandisäure;
Eisen-III-Komplex der 3,6,9-Triaza-3,6,9-tris(carboxymethyl)-4-(4-methoxybenzyl)-undecandisäure;
Wismutkomplex der 3,6,9-Triaza-3,6,9-tris(carboxymethyl)-4-(4-methoxybenzyl)-undecandisäure;
Gadoliniumkomplex der 3,6,9-Triaza-3,6,9-tris(carboxymethyl)-5-(4-methoxybenzyl)-undecandisäure;
Gadoliniumkomplex der 3,6,9-Triaza-3,6,9-tris(carboxymethyl)-4-[4-(4-methoxybenzyloxy)-benzyl]-undecandisäure;
Gadoliniumkomplex der 3,6,9-Triaza-3,6,9-tris(carboxymethyl)-4-benzyl-undecandisäure;
Ytterbiumkomplex der 3,6,9-Triaza-3,6,9-tris(carboxymethyl)-4-benzyl-undecandisäure;
Gadoliniumkomplex der 3,6,9-Triaza-3,6,9-tris(carboxymethyl)-4-benzyloxymethyl-undecandisäure;
Gadoliniumkomplex der 3,6,9-Triaza-3,6,9-tris(carboxymethyl)-4-(4-carboxymethoxybenzyl)-undecandisäure;
Gadoliniumkomplex der 3,6,9-Triaza-3,6,9-tris(carboxymethyl)-4-(4-ethoxybenzyl)-undecandisäure;
Europiumkomplex der 3,6,9-Triaza-3,6,9-tris(carboxymethyl)-4-(4-ethoxybenzyl)-undecandisäure;
Eisenkomplex der 3,6,9-Triaza-3,6,9-tris(carboxymethyl)-4-(4-ethoxybenzyl)-undecandisäure;
Gadoliniumkomplex der 3,6,9-Triaza-3,6,9-tris(carboxymethyl)-4-(4-butoxybenzyl)-undecandisäure;
Europiumkomlex der 3,6,9-Triaza-3,6,9-tris(carboxymethyl)-4-(4-butoxybenzyl)-undecandisäure;
Eisenkomplex der 3,6,9-Triaza-3,6,9-tris(carboxymethyl)-4-(4-butoxybenzyl)-undecandisäure;
Gadoliniumkomplex der 3,6,9-Triaza-3,6,9-tris(carboxymethyl)-4-(4-benzyloxybenzyl)-undecandisäure;
Europiumkomplex der 3,6,9-Triaza-3,6,9-tris(carboxymethyl)-4-(4-benzyloxybenzyl)-undecandisäure;
Eisenkomplex der 3,6,9-Triaza-3,6,9-tris(carboxymethyl)-4-(4-benzyloxybenzyl)-undecandisäure;

6. Pharmazeutische Mittel enthaltend mindestens eine physiologisch vertragliche Verbindung nach Anspruch 1 bis 5, gegebenenfalls mit den in der Galenik üblichen Zusätzen.

7. Verwendung von mindestens einer physiologisch verträglichen Verbindung gemäß Anspruch 1 für die Herstellung von Mitteln für die NMR- und Röntgen-Diagnostik und Strahlentherapie.

8. Verwendung von mindestens einer physiologisch verträglichen Verbindung gemäß Anspruch 1 bis 5 für die Herstellung von Mitteln für die NMR-Diagnostik des renalen und hepatobiliaren Systems.

9. Verwendung von mindestens einer physiologisch verträglichen Verbindung gemäß Anspruch 1 für die Herstellung von Mitteln für die NMR-Diagnostik des Magen-Darm-Traktes.

10. Verfahren zur Herstellung von Verbindungen der allgemeinen Formel I 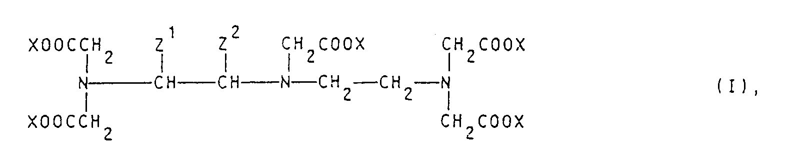 worin
Z¹ und Z² unabhängig voneinander für ein Wasserstoffatom oder den Rest -(CH₂)ₘ-(C₆H₄)_{q}-(O)ₖ-(CH₂)ₙ-(C₆H₄)ₗ-(O)ᵣ-R,
worin
m und n die Ziffern 0 - 20,
k, l, q und r die Ziffern 0 und 1 und
R ein Wasserstoffatom, einen gegebenenfalls OR¹-substituierten C₁-C₆-Alkylrest oder eine CH₂COOR¹-Gruppe mit R¹ in der Bedeutung eines Wasserstoffatoms, eines C₁-C₆-Alkylrestes oder einer Benzylgruppe bedeuten,
X für ein Wasserstoffatom und/oder ein Metallionenäquivalent eines Elements der Ordnungszahlen 21 - 29, 42, 44 oder 57 - 83 stehen, mit der Maßgabe, daß mindestens zwei der Substituenten X für ein Metallionenäquivalent stehen, daß einer der Substituenten Z¹ und Z² für ein Wasserstoffatom und der andere nicht für ein Wasserstoffatom steht, daß - wenn n und l jeweils für die Ziffer 0 stehen -
k und r nicht gleichzeitig jeweils die Ziffer 1 bedeuten, daß Z¹ oder Z² nicht für -CH₂-C₆H₄-O-CH₂-COOCH₂C₆H₅ oder -CH₂-C₆H₄-O-(CH₂)₅-COOCH₂C₆H₅ stehen, daß -(O)ᵣ-R nicht für -OH steht, daß Z¹ nicht für 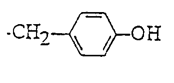 steht und daß die Summe von q und l die Ziffer 1 oder 2 ergibt, sowie deren Salze mit anorganischen und/oder organischen Basen, Aminosäuren oder Aminosäureamiden,
dadurch gekennzeichnet, daß man in an sich bekannter Weise eine Verbindung der allgemeinen Formel II 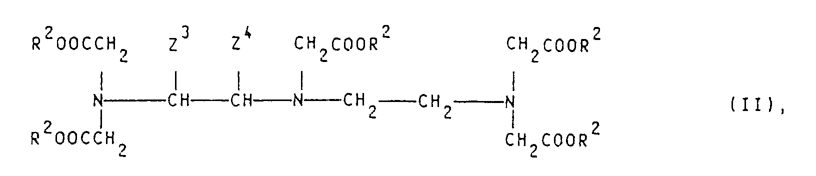 worin
R² für eine Säureschutzgruppe,
Z³ und Z⁴ jeweils für ein Wasserstoffatom oder den Rest -(CH₂)ₘ-(C₆H₄)_{q}-OH, mit der Maßgabe, daß einer der Substituenten Z³ und Z⁴ für ein Wasserstoffatom und der andere für den angegebenen Rest steht,
stehen,
in eine Verbindung mit dem für Z¹ und Z² angegebenen Rest umwandelt, die Säureschutzgruppen R² abspaltet, die so erhaltenen Komplexbildner-Säuren der allgemeinen Formel I' mit X in der Bedeutung eines Wasserstoffatoms mit mindestens einem Metalloxid oder Metallsalz eines Elements der Ordnungszahlen 21-29, 42, 44 oder 57-83 umsetzt und anschließend -falls gewünschtvorhandene acide Wasserstoffatome durch Kationen anorganischer und/oder organischer Basen, Aminosäuren oder Aminosäureamide substituiert.

11. Verfahren zur Herstellung der pharmazeutischen Mittel gemäß Anspruch 6, dadurch gekennzeichnet , daß man die in Wasser , physiologischer Salz- oder Proteinlösung gelöste oder suspendierte Komplexverbindung, gegebenenfalls mit den in der Galenik üblichen Zusätzen, in eine für die enterale oder parenterale Applikation geeignete Form bringt.

## Claims

1. Compounds of the general formula I 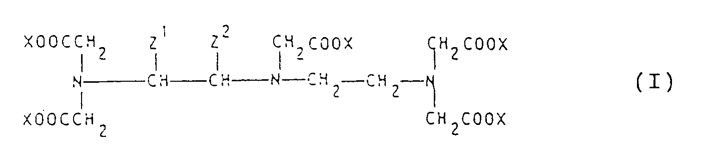 in which
Z¹ and Z² each independently of the other represent a hydrogen atom or the radical
-(CH₂)ₘ-(C₆H₄)_{q}-(O)ₖ-(CH₂)ₙ-(C₆H₄)ₗ-(O)ᵣ-R,
wherein
m and n represent the numbers 0 to 20,
k, l, q and r represent the numbers 0 and 1, and R represents a hydrogen atom, an optionally OR¹-substituted C₁-C₆alkyl radical or a group CH₂COOR¹ wherein R¹ represents a hydrogen atom, a C₁-C₆alkyl radical or a benzyl group,
and
X represents a hydrogen atom and/or a metal ion equivalent of an element of atomic numbers 21-29, 42, 44 or 57-83,
with the proviso that at least two of the X substituents represent a metal ion equivalent, that one of the substituents Z¹ and Z² represents a hydrogen atom and the other does not represent a hydrogen atom, that - when n and l each represent the number 0 - k and r do not at the same time each represent the number 1, that Z¹ or Z² does not represent -CH₂-C₆H₄-O-CH₂-COOCH₂C₆H₅ or -CH₂-C₆H₄-O-(CH₂)₅-COOCH₂C₆H₅, that -(O)ᵣ-R does not represent -OH, that Z¹ does not represent 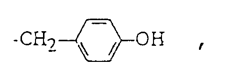 and that the sum of q and l gives the number 1 or 2, and their salts with inorganic and/or organic bases, amino acids or amino acid amides.

2. Compounds according to claim 1, characterised in that Z¹ represents a hydrogen atom and Z² represents the radical -(CH₂)ₘ-(C₆H₄)_{q}-(O)ₖ-(CH₂)ₙ-(C₆H₄)ₗ-(O)ᵣ-R.

3. Compounds according to claim 1, characterised in that Z² represents a hydrogen atom and Z¹ represents the radical -(CH₂)ₘ-(C₆H₄)_{q}-(O)ₖ-(CH₂)ₙ-(C₆H₄)ₗ-(O)ᵣ-R.

4. Compounds according to claim 1, characterised in that Z¹ or Z² represents the radicals -CH₂-C₆H₄-OCH₃, -CH₂-C₆H₅, -CH₂-C₆H₄-O-CH₂-C₆H₄-OCH₃, -CH₂-O-CH₂-C₆H₅, -CH₂-C₆H₄-O-CH₂-COOH, -CH₂-C₆H₄-OC₂H₅, -CH₂-C₆H₄-OC₄H₉, -CH₂-C₆H₄-O-CH₂-C₆H₅.

5. The compounds according to claim 1:
gadolinium complex of 3,6,9-triaza-3,6,9-tris(carboxymethyl)-4-(4-methoxybenzyl)-undecanedioic acid;
europium complex of 3,6,9-triaza-3,6,9-tris(carboxymethyl)-4-(4-methoxybenzyl)-undecanedioic acid;
iron(III) complex of 3,6,9-triaza-3,6,9-tris(carboxymethyl)-4-(4-methoxybenzyl)-undecanedioic acid;
bismuth complex of 3,6,9-triaza-3,6,9-tris(carboxymethyl)-4-(4-methoxybenzyl)-undecanedioic acid;
gadolinium complex of 3,6,9-triaza-3,6,9-tris(carboxymethyl)-5-(4-methoxybenzyl)-undecanedioic acid;
gadolinium complex of 3,6,9-triaza-3,6,9-tris(carboxymethyl)-4-[4-(4-methoxybenzyloxy)-benzyl]-undecanedioic acid;
gadolinium complex of 3,6,9-triaza-3,6,9-tris(carboxymethyl)-4-benzyl-undecanedioic acid;
ytterbium complex of 3,6,9-triaza-3,6,9-tris(carboxymethyl)-4-benzyl-undecanedioic acid;
gadolinium complex of 3,6,9-triaza-3,6,9-tris(carboxymethyl)-4-benzyloxymethyl-undecanedioic acid;
gadolinium complex of 3,6,9-triaza-3,6,9-tris(carboxymethyl)-4-(4-carboxymethoxybenzyl)-undecanedioic acid;
gadolinium complex of 3,6,9-triaza-3,6,9-tris(carboxymethyl)-4-(4-ethoxybenzyl)-undecanedioic acid;
europium complex of 3,6,9-triaza-3,6,9-tris(carboxymethyl)-4-(4-ethoxybenzyl)-undecanedioic acid;
iron complex of 3,6,9-triaza-3,6,9-tris(carboxymethyl)-4-(4-ethoxybenzyl)-undecanedioic acid;
gadolinium complex of 3,6,9-triaza-3,6,9-tris(carboxymethyl)-4-(4-butoxybenzyl)-undecanedioic acid;
europium complex of 3,6,9-triaza-3,6,9-tris(carboxymethyl)-4-(4-butoxybenzyl)-undecanedioic acid;
iron complex of 3,6,9-triaza-3,6,9-tris(carboxymethyl)-4-(4-butoxybenzyl)-undecanedioic acid;
gadolinium complex of 3,6,9-triaza-3,6,9-tris(carboxymethyl)-4-(4-benzyloxybenzyl)-undecanedioic acid;
europium complex of 3,6,9-triaza-3,6,9-tris(carboxymethyl)-4-(4-benzyloxybenzyl)-undecanedioic acid;
iron complex of 3,6,9-triaza-3,6,9-tris(carboxymethyl)-4-(4-benzyloxybenzyl)-undecanedioic acid

6. Pharmaceutical agents comprising at least one physiologically tolerable compound according to claims 1 to 5, optionally together with additives customary in galenic pharmacy.

7. Use of at least one physiologically tolerable compound according to claim 1 for the preparation of agents for NMR and X-ray diagnostics and radiotherapy.

8. Use of at least one physiologically tolerable compound according to claims 1 to 5 for the preparation of agents for NMR diagnostics of the renal and hepatobiliary system.

9. Use of at least one physiologically tolerable compound according to claim 1 for the preparation of agents for NMR diagnostics of the gastrointestinal tract.

10. Process for the preparation of compounds of the general formula I 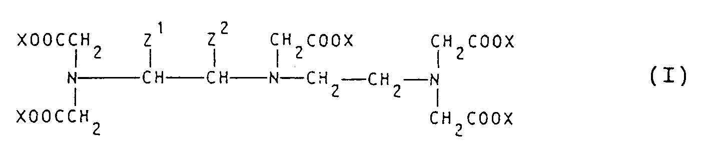 in which
Z¹ and Z² each independently of the other represent a hydrogen atom or the radical
-(CH₂)ₘ-(C₆H₄)_{q}-(O)ₖ-(CH₂)ₙ-(C₆H₄)ₗ-(O)ᵣ-R,
wherein
m and n represent the numbers 0 to 20,
k, l, q and r represent the numbers 0 and 1, and R represents a hydrogen atom, an optionally OR¹-substituted C₁-C₆alkyl radical or a group CH₂COOR¹ wherein R¹ represents a hydrogen atom, a C₁-C₆alkyl radical or a benzyl group,
and
X represents a hydrogen atom and/or a metal ion equivalent of an element of atomic numbers 21-29, 42, 44 or 57-83,
with the proviso that at least two of the X substituents represent a metal ion equivalent, that one of the substituents Z¹ and Z² represents a hydrogen atom and the other does not represent a hydrogen atom, that - when n and 1 each represent the number 0 - k and r do not at the same time each represent the number 1, that Z¹ or Z² does not represent -CH₂-C₆H₄-O-CH₂-COOCH₂C₆H₅ or -CH₂-C₆H₄-O-(CH₂)₅-COOCH₂C₆H₅, that -(O)ᵣ-R does not represent -OH, that Z¹ does not represent 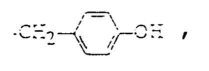 and that the sum of q and l gives the number 1 or 2, and their salts with inorganic and/or organic bases, amino acids or amino acid amides,
characterised in that a compound of the general formula II 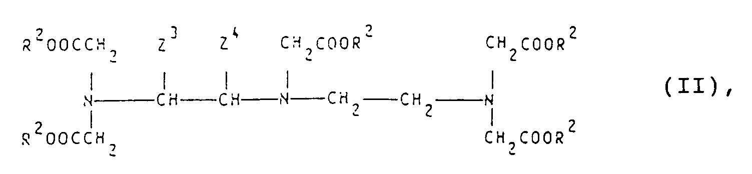 in which
R² represents an acid protecting group, and
Z³ and Z⁴ each represent a hydrogen atom or the radical -(CH₂)ₘ-(C₆H₄)_{q}-OH, with the proviso that one of the substituents Z³ and Z⁴ represents a hydrogen atom and the other represents the mentioned radical,
is converted in a manner known per se into a compound containing the radical mentioned for Z¹ and Z², the acid protecting groups R² are removed, the resulting complexing agent acids of the general formula I' wherein X represents a hydrogen atom are reacted with at least one metal oxide or metal salt of an element of atomic numbers 21-29, 42, 44 or 57-83, and then - if desired - acidic hydrogen atoms that are present are replaced by cations of inorganic and/or organic bases, amino acids or amino acid amides.

11. Process for the preparation of the pharmaceutical agents according to claim 6, characterised in that the complex compound dissolved or suspended in water, physiological saline solution or physiological protein solution, optionally together with additives customary in galenic pharmacy, is brought into a form suitable for enteral or parenteral administration.

## Revendications

1. Composés de formule générale I 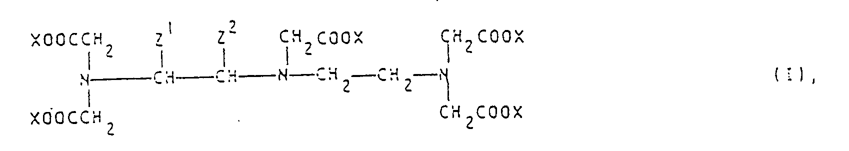 dans laquelle Z¹ et Z², indépendamment l'un de l'autre, représentent un atome d'hydrogène ou un radical
-(CH₂)ₘ-(C₆H₄)_{q}-(O)ₖ-(CH₂)ₙ-(C₆H₄)ₗ-(O)ᵣ-R,
où
m et n représentent les nombres de 0 à 20,
k, l, q et r représentent les nombres 0 et 1, et
R représente un atome d'hydrogène, un radical alkyle en C₁-C₆ éventuellement substitué par OR¹ ou un groupe CH₂COOR¹ avec R¹ ayant la signification d'un atome d'hydrogène, d'un radical akyle en C₁-C₆ ou d'un groupe benzyle,
X représente un atome d'hydrogène et/ou un équivalent d'ion métallique d'un élément ayant le numéro atomique 21 à 29, 42, 44 ou 57 à 83, à la condition, qu'au moins deux des substituants X représentent un équivalent d'ion métallique, que au moins l'un des substituants Z¹ et Z² représente un atome d'hydrogène et que l'autre ne représente pas un atome d'hydrogène, que - lorsque n et l représentent respectivement le nombre 0 -,
k et r ne signifient pas simultanément le nombre 1, que Z¹ ou Z² ne représentent pas
-CH₂-C₆H₄-O-CH₂-COOCH₂C₆H₅ ou -CH₂-C₆H₄-O-(CH₂)₅-COOCH₂C₆H₅
que -(O)ᵣ-R ne représente pas -OH, que Z¹ ne représente pas 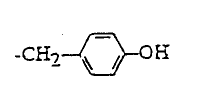 et que la somme de q et l donne les nombres 1 ou 2, ainsi que leurs sels avec des bases minérales et/ou organiques, acides aminés ou amides d'acides aminés.

2. Composés selon la revendication 1 caractérisés en ce que Z¹ représente un atome d'hydrogène et Z² représente le radical

3. Composés selon la revendication 1 caractérises en ce que Z² représente un atome d'hydrogène et Z¹ représente le radical

4. Composés selon la revendication 1 caractérises en ce que Z¹ ou Z² représentent les radicaux

5. Composés selon la revendication 1 :
complexe de gadolinium du diacide 3,6,9-triaza-3,6,9-tris-(carboxyméthyl)-4-(4-méthoxybenzyl)-undécanoïque,
complexe d'europium du diacide 3,6,9-triaza-3,6,9-tris-(carboxyméthyl)-4-(4-méthoxybenzyl)-undécanoïque,
complexe de fer(III) du diacide 3,6,9-triaza-3,6,9-trls-(carboxyméthyl)-4-(4-méthoxybenzyl)-undécanoïque,
complexe de tungstène du diacide 3,6,9-triaza-3,6,9-tris-(carboxyméthyl)-4-(4-méthoxybenzyl)-undécanoïque,
complexe de gadolinium du diacide 3,6,9-triaza-3,6,9-tris-(carboxyméthyl)-5-(4-méthoxybenzyl)-undécanoïque,
complexe de gadolinium du diacide 3,6,9-triaza-3,6,9-tris-(carboxyméthyl)-4-[4-(4-méthoxybenzyloxy)-benzyl]-undécanoïque,
complexe de gadolinium du diacide 3,6,9-triaza-3,6,9-tris-(carboxyméthyl)-4-benzyl-undécanoïque,
complexe d'ytterbium du diacide 3,6,9-triaza-3,6,9-tris-(carboxyméthyl)-4-benzyl-undécanoïque,
complexe de gadolinium du diacide 3,6,9-triaza-3,6,9-tris-(carboxyméthyl)-4-benzyloxyméthyl-undécanoïque,
complexe de gadolinium du diacide 3,6,9-triaza-3,6,9-tris-(carboxyméthyl)-4-(4-carboxyméthoxybenzyl)-undécanoïque,
complexe de gadolinium du diacide 3,6,9-triaza-3,6,9-tris-(carboxyméthyl)-4-(4-éthoxybenzyl)-undécanoïque,
complexe d'europium du diacide 3,6,9-triaza-3,6,9-tris-(carboxyméthyl)-4-(4-éthoxybenzyl)-undécanoïque,
complexe de fer du diacide 3,6,9-triaza-3,6,9-tris-(carboxyméthyl)-4-(4-éthoxybenzyl)-undécanoïque,
complexe de gadolinium du diacide 3,6,9-triaza-3,6,9-tris-(carboxyméthyl)-4-(4-butoxybenzyl)-undécanoïque,
complexe d'europium du diacide 3,6,9-triaza-3,6,9-tris-(carboxyméthyl)-4-(4-butoxybenzyl)-undécanoïque,
complexe de fer du diacide 3,6,9-triaza-3,6,9-tris-(carboxyméthyl)-4-(4-butoxybenzyl)-undécanoïque,
complexe de gadolinium du diacide 3,6,9-triaza-3,6,9-tris-(carboxyméthyl)-4-(4-benzyloxybenzyl)-undécanoïque,
complexe d'europium du diacide 3,6,9-triaza-3,6,9-tris-(carboxyméthyl)-4-(4-benzyloxybenzyl)-undécanoïque,
complexe de fer du diacide 3,6,9-triaza-3,6,9-tris-(carboxyméthyl)-4-(4-benzyloxybenzyl)-undécanoïque.

6. Agents pharmaceutiques contenant au moins un composé physiologiquement compatible selon la revendication 1 à 5, éventuellement avec des adjuvants usuels en galénique.

7. Utilisation d'au moins un composé physiologiquement compatible selon la revendication 1, pour la préparation d'agents pour le diagnostic par RMN et pour le radiodiagnostic et pour la thérapie par rayonnement.

8. Utilisation d'au moins un composé physiologiquement compatible selon la revendication 1 à 5, pour la préparation d'agents pour le diagnostic par RMN du système rénal et hépatobiliaire.

9. Utilisation d'au moins un composé physiologiquement compatible selon la revendication 1 à 5, pour la préparation d'agents pour le diagnostic par RMN du tractus gastro-intestinal.

10. Procédé pour la préparation du composé de formule générale I 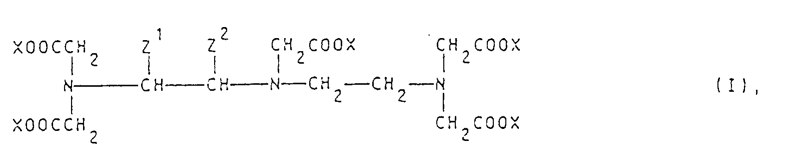 dans laquelle Z¹ et Z², indépendamment l'un de l'autre, représentent un atome d'hydrogène ou un radical
-(CH₂)ₘ-(C₆H₄)_{q}-(O)ₖ-(CH₂)ₙ-(C₆H₄)ₗ-(O)ᵣ-R,
où
m et n représentent les nombres de 0 à 20,
k, l, q et r représentent les nombres 0 et 1, et
R représente un atome d'hydrogène, un radical alkyle en C₁-C₆ éventuellement substitué par OR¹ ou un groupe CH₂COOR¹ avec R¹ ayant la signification d'un atome d'hydrogène, d'un radical akyle en C₁-C₆ ou d'un groupe benzyle,
X représente un atome d'hydrogène et/ou un équivalent d'ion métallique d'un élément ayant le numéro atomique 21 à 29, 42, 44 ou 57 à 83, à la condition, qu'au moins deux des substituants X représentent un équivalent d'ion métallique, que au moins l'un des substituants Z¹ et Z² représente un atome d'hydrogène et que l'autre ne représente pas un atome d'hydrogène, que - lorsque n et l représentent respectivement le nombre 0 -,
k et r ne signifient pas simultanément le nombre 1, que Z¹ ou Z² ne représentent pas
-CH₂-C₆H₄-O-CH₂-COOCH₂C₆H₅ ou -CH₂-C₆H₄-O-(CH₂)₅-COOCH₂C₆H₅
que -(O)ᵣ-R ne représente pas -OH, que Z¹ ne représente pas 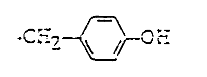 et que la somme de q et l donne les nombres 1 ou 2, ainsi que leurs sels avec des bases minérales et/ou organiques, acides aminés ou amides d'acides aminés, caractérisés en ce qu'on transforme de façon connue en soi un composé de formule générale II 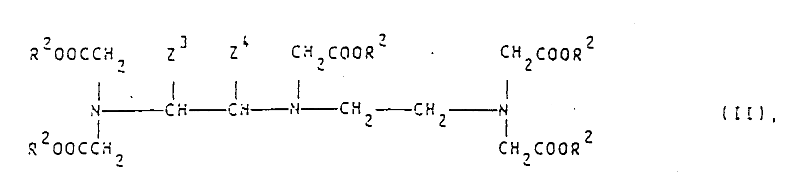 dans laquelle
R² représente un groupe protecteur d'acide,
Z³ et Z⁴ représente chaque fois un atome d'hydrogène ou un radical -(CH₂)ₘ-(C₆H₄)_{q}-OH, à la condition que l'un des substituants Z³ et Z⁴ représente un atome d'hydrogène et que l'autre représente le radical donné, en un composé comportant le radical indiqué pour Z¹ et Z², on élimine le groupe protecteur d'acide R², on fait réagir les acides compexants ainsi obtenus de formule générale I' avec X ayant la signification d'un atome d'hydrogène avec au moins un oxyde métallique ou sel métallique d'un élément ayant des numéros atomiques de 21 à 29, 42, 44 ou 57 à 83 et finalement - si on le désire - on effectue la substitution des atomes d'hydrogène acides présents par des cations de bases minérales et/ou organiques, d'acides aminés ou d'amides d'acides aminés.

11. Procédé pour la préparation d'agents pharmaceutiques selon la revendication 6, caractérisé en ce qu'on met en forme appropriée le composé complexe suspendu ou dissout dans l'eau, solution physiologique ou solution de protéines, éventuellement avec des adjuvants usuels en galénique, pour une administration entérale ou parentérale.
